# EUROPÄISCHE PATENTSCHRIFT

(11) **EP 3 389 791 B1**
(45) Veröffentlichungstag und Bekanntmachung des Hinweises auf die Patenterteilung: **05.02.2020**
(21) Anmeldenummer: 16809043.9
(22) Anmeldetag: 09.12.2016
(51) Int. Cl.: A61Q 5/10, A61K 8/22, A61K 8/362, A61K 8/42, A61K 8/44, A61K 8/46

(54) **OXIDATIONSMITTEL ZUR OXIDATIVEN FÄRBE- UND BLONDIERBEHANDLUNG KERATINISCHER FASERN MIT REDUZIERTER SCHÄDIGUNG**
OXIDIZING AGENT FOR OXIDATIVELY COLORING OR BLEACHING KERATINIC FIBERS WITH DECREASED DAMAGEING
AGENT OXYDATIF POUR COLORER OU ÉCLAIRCIR AVEC MOINDRE DÉGRADATION LES FIBRES KÉRATINIQUES

(30) Priorität: 14.12.2015 DE 102015225137
(43) Veröffentlichungstag der Anmeldung: 24.10.2018
(73) Patentinhaber: Henkel AG & Co. KGaA, 40589 Düsseldorf (DE)
(72) Erfinder: MANNECK, Hartmut, 23858 Barnitz (DE); HIPPE, Thomas, 25482 Appen (DE); HOEPFNER, Stefan, 22523 Hamburg (DE); BRENDER, Jessica, 22763 Hamburg (DE)
(86) Internationale Anmeldenummer: PCT/EP2016/080408
(87) Internationale Veröffentlichungsnummer: WO 2017/102582

(56) Entgegenhaltungen:
- EP-A1- 2 749 322
- EP-A2- 2 295 027
- WO-A1-2015/028017

## Beschreibung

Die vorliegende Erfindung betrifft ein Haar schonendes Oxidationsmittel zur oxidativen Haarfärbung oder Blondierung sowie ein schonendes Verfahren zur oxidativen Haarfärbung oder Blondierung, bei dem keratinische Fasern vor oxidativen Einflüssen geschützt bzw. oxidative Haarschäden repariert werden.

Beim oxidativen Färben oder Blondieren von Haaren tritt das Problem auf, dass es aufgrund der aggressiven Agentien zu Schäden an der keratinischen Faser kommen kann. Insbesondere die natürliche Hydrophobizität der keratinischen Faser wird reduziert, da die Färbe- bzw. Aufhellmittel das Haar zunächst penetrationsfähig machen müssen, um ihre Wirkung zu entfalten. Die Wasser abweisende Wirkung ist aber einerseits ein natürlicher Schutz des Haares, andererseits sind vom Verbraucher erwünschte Parameter wie Glanz, Geschmeidigkeit, Griff und "Fallen" der Haare eng mit ihr verknüpft.

Um die genannten Nachteile zu überwinden, sind so genannte Vorbehandlungsmittel auf dem Markt, die das Haar vor dem aggressiven Einfluss schützen sollen. Diese beschweren das Haar aber oft oder beeinträchtigen den Erfolg der im Anschluss stattfindenden Aufhellung bzw. Färbung des Haares; insbesondere die Waschechtheit der Färbung kann durch das Vorbehandlungsmittel verschlechtert sein. Auch sind zahlreiche Nachbehandlungsmittel bekannt, mit denen versucht wird, die bei der oxidativen Färbebehandlung verursachten Haarschädigungen zu reparieren. Alle diese Verfahren erfordern aber ein mehrstufiges Anwendungsverfahren, eben entweder eine dem Färben vor- oder nachgelagerte Applikation eines weiteren Haarbehandlungsmittels. Dies wird vom Verbraucher häufig als lästig wahrgenommen, da bereits die oxidative Färbebehandlung selbst mit mehreren Arbeitsschritten und einer Einwirkzeit von bis zu 60 Minuten sehr aufwändig ist.

Der vorliegenden Erfindung lag die Aufgabe zugrunde, ein Mittel und ein Verfahren zur oxidativen Haarfärbung mit einer Haar schützenden Behandlung bereitzustellen, das die genannten Nachteile überwindet, ohne das Farbergebnis der oxidativen Färbebehandlung negativ zu beeinflussen. Dabei sollten insbesondere ein Färbemittel und ein Verfahren bereitgestellt werden, bei dem das Haar nicht beschwert wird und eine möglichst geringe Haarschädigung auftritt. Weiterhin sollte der erzielte Haarschutz möglichst wenig zeitaufwändig sein und möglichst zusammen mit dem Färbeschritt selbst erfolgen.

Der Einsatz von Dicarbonsäuren wie Maleinsäure oder Bernsteinsäure in der Haarpflege ist Stand der Technik. Diese werden in Shampoos und insbesondere in Conditionern breit eingesetzt, um dort Pflegeeffekte zu entfalten. So offenbart die Patentanmeldung WO 2005/115314A1 ein Verfahren zur Restrukturierung keratinischer Fasern, bei dem die Keratinfasern mit Cystin und mit mindestens einer Dicarbonsäure mit 2 bis 10 Kohlenstoffatomen in Kontakt gebracht werden, wobei bevorzugte Dicarbonsäuren ausgewählt sind aus Oxalsäure, Malonsäure, Bernsteinsäure, Glutarsäure, Adipinsäure, Pimelinsäure, Azelainsäure, Maleinsäure, Fumarsäure und Sorbinsäure und Bernsteinsäure besonders bevorzugt ist. Die Patentanmeldung DE 10051774 A1 beschreibt die Verwendung kurzkettiger Carbonsäuren mit einem Molekulargewicht unter 750 g/mol in kosmetischen Mitteln als Wirkstoff zur Restrukturierung keratinischer Fasern. Die Patentanmeldung EP1174112A offenbart Haarbehandlungsmittel, die neben einer organischen Säure als weitere zwingende Bestandteile ein organisches Lösungsmittel, ein kationisches Tensid und einen höheren Alkohol enthalten und zur Reparatur von Poren in Haaren dienen.

In der Mintel-Datenbank sind unter den Eintragsnummern 529647, 2061070, 743114, 1431193 und 406342 oxidative Haarfärbe- oder Blondierungsmittel-Kits offenbart, die eine wässrige Wasserstoffperoxidzubereitung umfassen, die Maleinsäure enthält. Keine dieser Wasserstoffperoxidzubereitungen enthält eine Aminosäure.

Es wurde nun gefunden, dass oxidative Behandlungsmittel, die neben typischen Bestandteilen, wie Wasserstoffperoxid und Wasser, mindestens eine Dicarbonsäure mit 2 bis 10 Kohlenstoffatomen und mindestens eine Aminosäure, bevorzugt mindestens eine Aminosäure der Formel (VI), enthalten, zu deutlich verbessertem Haarschutz bei der oxidativen Haarbehandlung führen, ohne dass die Ergebnisse der oxidativen Färbe- oder Blondierbehandlung beeinträchtigt werden. Es wurde überraschend gefunden, dass durch den Gehalt an mindestens einer Dicarbonsäure mit 2 bis 10 Kohlenstoffatomen in Kombination mit mindestens einer Aminosäure, bevorzugt mindestens einer Aminosäure der Formel (VI), das Haar während der Färbens und/oder des Aufhellens vor Schädigungen durch den hohen pH des Mittels und durch das Oxidationsmittel geschützt wird. Dies ließ sich unter anderem daran feststellen, dass beim anschließenden Kämmen weniger Haarbruch auftrat und das Haar weniger an Elastizität einbüßte, wie sich durch Zug-Dehn-Messungen nachweisen ließ, als nach der Applikation nicht erfindungsgemäßer Färbe- und Blondiermittel.

Gegenstand der vorliegenden Erfindung ist in einer ersten Ausführungsform ein oxidatives Behandlungsmittel für keratinische Fasern, insbesondere für Humanhaar, enthaltend
a) mindestens eine Dicarbonsäure mit 2 bis 10 Kohlenstoffatomen und/oder mindestens ein Salz dieser Säure(n),
b) mindestens eine Aminosäure,
c) weiterhin Wasserstoffperoxid und
d) Wasser,
wobei das oxidative Behandlungsmittel einen pH-Wert im Bereich von 2,5 - 6,5, bevorzugt im Bereich von 3 - 5,5, besonders bevorzugt im Bereich von 3,5 bis 5,0, aufweist, jeweils bei 20°C gemessen.

Ein weiterer Gegenstand der vorliegenden Erfindung ist ein Verfahren zur oxidativen Färbung und/oder Aufhellung keratinischer Fasern, insbesondere von Humanhaar, bei dem ein oxidatives Behandlungsmittel auf die keratinischen Fasern, insbesondere auf das Humanhaar, aufgetragen und ggf. nach einer Einwirkzeit von 0,1 bis 60 Minuten, bevorzugt 1 bis 50 Minuten, besonders bevorzugt 10 bis 45 Minuten, außerordentlich bevorzugt 30 bis 45 Minuten, wieder ausgespült wird, wobei dieses Behandlungsmittel
a) mindestens eine Dicarbonsäure mit 2 bis 10 Kohlenstoffatomen und/oder mindestens ein Salz dieser Säure(n),
b) mindestens eine Aminosäure,
c) Wasserstoffperoxid und
d) Wasser
enthält, wobei das oxidative Behandlungsmittel einen pH-Wert im Bereich von 2,5 - 6,5, bevorzugt im Bereich von 3 - 5,5, besonders bevorzugt im Bereich von 3,5 bis 5,0, aufweist, jeweils bei 20°C gemessen.

Ein weiterer Gegenstand der vorliegenden Erfindung ist ein Verfahren zur oxidativen Färbung und/oder Aufhellung keratinischer Fasern, insbesondere von Humanhaar, das folgende Verfahrensschritte umfasst:
I. Bereitstellen eines oxidativen Behandlungsmittels (A), enthaltend
   a) mindestens eine Dicarbonsäure mit 2 bis 10 Kohlenstoffatomen und/oder mindestens ein Salz dieser Säure(n), die bevorzugt ausgewählt ist aus Maleinsäure, Fumarsäure, Bernsteinsäure, Äpfelsäure, Oxalsäure, Malonsäure, Adipinsäure, Pimelinsäure, Korksäure, Azelainsäure, Sebacinsäure, D-Weinsäure, L-Weinsäure, meso-Weinsäure, Traubensäure, alpha-Ketoglutarsäure, beta-Ketoglutarsäure sowie Mischungen dieser Säuren, besonders bevorzugt ausgewählt aus Bernsteinsäure, Äpfelsäure, Maleinsäure und Fumarsäure, außerordentlich bevorzugt ausgewählt aus Bernsteinsäure, weiterhin
   b) mindestens eine Aminosäure, die bevorzugt ausgewählt ist aus Aminosäuren der Formel (VI) 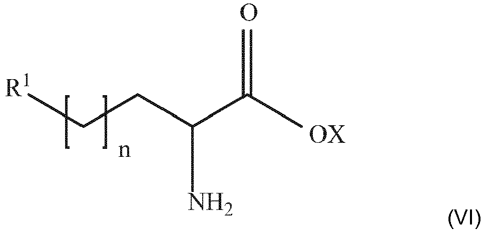 worin
      X für ein Wasserstoffatom oder ein ein- oder zweiwertiges Kation steht;
      n für null, 1, 2 oder 3 steht;
      R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens ein Salz dieser Aminosäure,
   c) Wasserstoffperoxid und
   d) Wasser,
   e) wobei das oxidative Behandlungsmittel einen pH-Wert im Bereich von 2,5 - 6,5, bevorzugt im Bereich von 3 - 5,5, besonders bevorzugt im Bereich von 3,5 bis 5,0, aufweist, jeweils bei 20°C gemessen,
   f) optional weiterhin mindestens eine Substanz, die ausgewählt ist aus
      - Verbindungen der allgemeinen Formel (III), 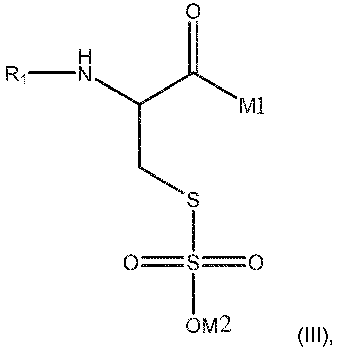 wobei
         R1 für ein Wasserstoffatom oder für ein Strukturelement der Formel (IV) steht 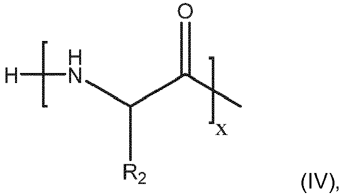 wobei
         x für eine ganze Zahl von 1 bis 100 steht,
         der Rest R2 in jedem der Strukturelemente der Formel (IV) jeweils unabhängig vom vorangegangenen Strukturelement der Formel (IV) gewählt werden kann,
         R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)-methyl-Gruppe,
         M1 steht für die Gruppierung -OM2 oder für ein Strukturelement der Formel (V) 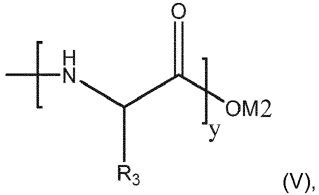 wobei
            y für eine ganze Zahl von 1 bis 100 steht,
            der Rest R3 in jedem der Strukturelemente der Formel (V) jeweils unabhängig vom vorangegangenen Strukturelement der Formel (V) gewählt werden kann,
            R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)-methyl-Gruppe,
            M2 steht für ein Wasserstoffatom, ein Äquivalent eines ein oder mehrwertigen Kations oder ein Ammoniumion (NH₄)⁺, und
      - mindestens einem Chelatbildner, bevorzugt in einer Gesamtmenge von 0,01 - 2,5 Gew.-%, besonders bevorzugt in einer Gesamtmenge von 0,1 - 1,5 Gew.-%, außerordentlich bevorzugt in einer Gesamtmenge von 0,2 - 0,6 Gew.-%, jeweils bezogen auf das Gewicht des oxidativen Behandlungsmittels (A), und
      - mindestens einem Fettsäureamid mit der Formel (AMID-I), bevorzugt in einer Gesamtmenge von 0,1 bis 10 Gew.-%, besonders bevorzugt 0,2 bis 5 Gew.-%, außerordentlich bevorzugt 0,4 - 2 Gew.-%, jeweils bezogen auf das Gewicht des oxidativen Behandlungsmittels (A), 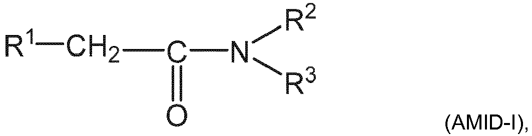 worin
         R¹ ausgewählt ist aus linearen und verzweigten C4-C22-Alkylgruppen, die gesättigt oder ungesättigt sind, wobei die Alkyl- und Alkenylgruppen unsubstituiert sind oder mit Sauerstoffatomen, Stickstoffatomen, Hydroxylgruppen, Ethergruppen, Oxyalkylengruppen, Polyoxyalkylengruppen, Carbonsäuregruppen, Amingruppen, Amidgruppen, Halogen-enthaltenden Gruppen, Estergruppen, Siloxangruppen oder Polysiloxangruppen substituiert sein können,
         und alkoxylierten Alkylgruppen der Formeln: 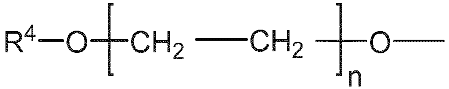 und 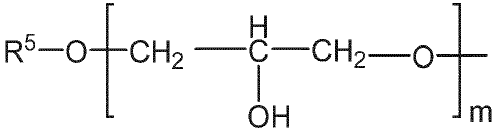 worin:
         R⁴ und R⁵, die gleich oder verschieden sein können, ausgewählt sind aus linearen und verzweigten C4-C22-Alkylgruppen, die gesättigt oder ungesättigt sind,
         n eine ganze Zahl im Bereich von 1 bis 10, bevorzugt im Bereich von 1 bis 4 ist und
         m eine ganze Zahl im Bereich von 1 bis 6 liegt und
         R² und R³, die gleich oder verschieden sein können, jeweils unabhängig voneinander ausgewählt sind aus H; linearen und verzweigten C1-C22-Alkylgruppen, die gesättigt oder ungesättigt sind, wobei die Alkyl- und Alkenylgruppen unsubstituiert sind oder mit Sauerstoffatomen, Stickstoffatomen, Hydroxylgruppen, Ethergruppen, Oxyalkylengruppen, insbesondere Oxyethylengruppen, Polyoxyalkylengruppen, insbesondere Polyoxyethylengruppen, Carbonsäuregruppen, Amingruppen, Alkylamingruppen, insbesondere C1-C3-Alkylamingruppen, Dialkylamingruppen, insbesondere Di-(C1-C3)-alkylamingruppen, substituierten und nicht substituierten 4,5-Dihydroimidazoliumgrupppen, Amidgruppen, Halogen enthaltenden Gruppen, Estergruppen, Siloxangruppen oder Polysiloxangruppen substituiert sind,
II. Bereitstellen einer Zusammensetzung (B), enthaltend
   g) mindestens ein Alkalisierungsmittel, ausgewählt aus Ammoniumhydroxid, Monoethanolamin und Natriumsilikaten sowie Mischungen hiervon,
   h) ggf. Wasser und
   i) ggf. mindestens ein Oxidationsfarbstoffvorprodukt und/oder mindestens einen direktziehenden Farbstoff,
III. Vermischen der Zusammensetzungen (A) und (B) miteinander, wobei das Gewichtsverhältnis von (A) zu (B) bevorzugt im Bereich von 10:1 bis 1:4, bevorzugt im Bereich von 5:1 bis 1:3, besonders bevorzugt im Bereich von 4:1 bis 1:2, außerordentlich bevorzugt im Bereich von 2:1 bis 1:1 liegt, direkt anschließend
IV. Auftragen der Mischung aus (A) und (B) auf die keratinischen Fasern, insbesondere auf das Humanhaar, und
V. Ausspülen nach einer Einwirkzeit von 0,1 bis 60 Minuten, bevorzugt 1 bis 50 Minuten, besonders bevorzugt 10 bis 45 Minuten, außerordentlich bevorzugt 30 bis 45 Minuten,
VI. ggf. weitere Haarbehandlungen, wie Verformen, Konditionieren und/oder Trocknen.

Unter keratinischen Fasern, keratinhaltigen Fasern oder Keratinfasern sind bevorzugt menschliche Haare zu verstehen, weiterhin Pelze, Wolle und Federn.

Unter "direkt anschließend" wird erfindungsgemäß ein Zeitraum von 1 bis 600 Sekunden verstanden.

### Dicarbonsäuren mit 2 bis 10 Kohlenstoffatomen und/oder mindestens ein Salz dieser Säure(n)

Erfindungsgemäß bevorzugte Dicarbonsäuren mit 2 bis 10 Kohlenstoffatomen sind ausgewählt aus Maleinsäure, Fumarsäure, Bernsteinsäure, Äpfelsäure, Oxalsäure, Malonsäure, Adipinsäure, Pimelinsäure, Korksäure, Azelainsäure, Sebacinsäure, D-Weinsäure, L-Weinsäure, meso-Weinsäure, Traubensäure, alpha-Ketoglutarsäure, beta-Ketoglutarsäure sowie Mischungen dieser Säuren.

Erfindungsgemäß besonders bevorzugt sind Maleinsäure, Fumarsäure, Äpfelsäure und Bernsteinsäure, sowie Mischungen dieser Säuren. Erfindungsgemäß außerordentlich bevorzugt ist Bernsteinsäure. Die genannten Dicarbonsäuren leisten einen wesentlichen Beitrag zur verringerten Haarschädigung durch die erfindungsgemäßen Behandlungsmittel.

Je nach pH-Wert des erfindungsgemäßen oxidativen Behandlungsmittels oder der in einem der erfindungsgemäßen Färbe- oder Blondierverfahren eingesetzten Zusammensetzung (B) kann die mindestens eine Dicarbonsäure mit 2 bis 10 Kohlenstoffatomen als undissoziierte Säure, teilweise dissoziiert oder vollständig dissoziiert vorliegen. Liegt die mindestens eine Dicarbonsäure mit 2 bis 10 Kohlenstoffatomen teilweise dissoziiert oder vollständig dissoziiert vor, so ist das Gegenion ausgewählt aus physiologisch verträglichen Kationen, wie insbesondere den Alkalimetall-, Erdalkalimetall- und Zinkionen sowie Ammoniumionen, Alkylammonium-, Alkanolammonium- und Glucammoniumionen, insbesondere die Mono-, Di- und Trimethyl-, -ethyl- und -hydroxyethylammoniumionen. Ebenfalls bevorzugt sind die Salze der Dicarbonsäuren mit 2 bis 10 Kohlenstoffatomen mit Amino-C₁-C₆-Alkanolen, insbesondere mit Monoethanolamin, und Amino-C₁-C₆-Alkandiolen, insbesondere mit 2-Amino-2-methylpropan-1-ol, 2-Amino-2-methylpropan-1,3-diol, 2-Aminopropan-1-ol, 3-Aminopropan-1-ol, 1-Aminopropan-2-ol (MIPA) und 2-Amino-2-(hydroxymethyl)propan-1,3-diol (TRIS), wobei die Salze mit Monoethanolamin, 2-Amino-2-methylpropan-1-ol und 2-Amino-2-methylpropan-1,3-diol besonders bevorzugt sind.

Außerordentlich bevorzugt sind Natrium-, Kalium-, Magnesium-, Ammonium und Monoethanolammonium-lonen als Gegenionen für die teilweise oder vollständig dissoziierten Dicarbonsäuren mit 2 bis 10 Kohlenstoffatomen. Daneben können jedoch auch mit alkalisch reagierenden Aminosäuren, wie beispielsweise Arginin, Lysin, Ornithin und Histidin, neutralisierte Dicarbonsäuren mit 2 bis 10 Kohlenstoffatomen eingesetzt werden.

Die Natrium-, Kalium-, Ammonium-, Monoethanolammonium-, Lysin- sowie Argininsalze sowie deren Mischungen sind bevorzugte Salze der Dicarbonsäuren mit 2 bis 10 Kohlenstoffatomen.

Bevorzugte erfindungsgemäße Behandlungsmittel enthalten die mindestens eine Dicarbonsäure mit 2 bis 10 Kohlenstoffatomen oder ein oder mehrere Salze hiervon in einer Gesamtmenge von 0,2 bis 4 Gew.-%, bevorzugt 0,33 bis 3 Gew.-%, besonders bevorzugt 0,5 bis 2 Gew.-%, jeweils umgerechnet auf die undissoziierte Säure und bezogen auf das Gewicht des Behandlungsmittels.

Auch wenn die Dicarbonsäuren in Salzform vorliegen, beziehen sich die vorstehenden Mengenangaben auf die jeweilige Dicarbonsäure in undissoziierter Form, um die Mengenangabe nicht durch unterschiedliche Molgewichte der Salze zu verfälschen. Eine Einwaage von 15 Gew.-% Dinatriumsuccinathexahydrat ergäbe beispielsweise eine Konzentration an Bernsteinsäure von umgerechnet 6,55 Gew.-%.

### Aminosäuren

Die verringerte Haarschädigungswirkung der erfindungsgemäßen Behandlungsmittel ist wesentlich zurückzuführen auf die mindestens eine vorgenannte Dicarbonsäure im Zusammenwirken mit mindestens einer Aminosäure.

Die erfindungsgemäßen Behandlungsmittel enthalten daher als weitere obligatorische Komponente mindestens eine Aminosäure. Bevorzugte Aminosäuren sind ausgewählt aus Arginin, Lysin, Histidin, Asparagin, Glutamin, Cystein, Methionin, Tryptophan, Serin, Alanin, Asparaginsäure, Glutaminsäure, Glycin, Isoleucin, Leucin, Phenylalanin, Prolin, Threonin, Tyrosin und Valin sowie Mischungen dieser Aminosäuren. Weitere erfindungsgemäß bevorzugte oxidative Behandlungsmittel enthalten mindestens eine Aminosäure in einer Gesamtmenge von 0,05 bis 3 Gew.-%, bevorzugt 0,1 bis 2 Gew.-%, besonders bevorzugt 0,2 bis 1,2 Gew.-%, jeweils umgerechnet auf die undissoziierte Säure und bezogen auf das Gewicht des oxidativen Behandlungsmittels. Weitere erfindungsgemäß bevorzugte oxidative Behandlungsmittel enthalten mindestens eine Aminosäure, ausgewählt aus Arginin, Lysin, Histidin, Asparagin, Glutamin, Cystein, Methionin, Tryptophan, Serin, Alanin, Asparaginsäure, Glutaminsäure, Glycin, Isoleucin, Leucin, Phenylalanin, Prolin, Threonin, Tyrosin und Valin sowie Mischungen dieser Aminosäuren, in einer Gesamtmenge von 0,05 bis 3 Gew.-%, bevorzugt 0,1 bis 2 Gew.-%, besonders bevorzugt 0,2 bis 1,2 Gew.-%, jeweils umgerechnet auf die undissoziierte Säure und bezogen auf das Gewicht des oxidativen Behandlungsmittels.

### Aminosäure der Formel (VI)

Eine besonders stark verringerte Haarschädigungswirkung der erfindungsgemäßen Behandlungsmittel ist wesentlich zurückzuführen auf die vorgenannten Dicarbonsäuren im Zusammenwirken mit mindestens einer ausgewählten Aminosäure der Formel (VI).

Bevorzugte erfindungsgemäße oxidative Behandlungsmittel enthalten daher als obligatorische Komponente mindestens eine Aminosäure der Formel (VI) 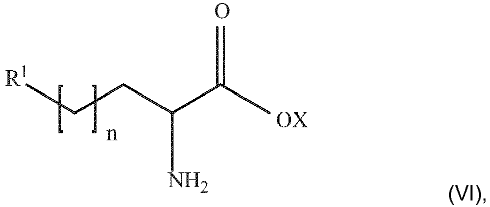 worin
X für ein Wasserstoffatom oder ein ein- oder zweiwertiges Kation steht;
n für null, 1, 2 oder 3 steht;
R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens einem Salz dieser Aminosäure.

Bevorzugte Aminosäuren der Formel (VI) sind ausgewählt aus Arginin, Lysin, Histidin, Asparagin, Glutamin, Cystein, Methionin, Tryptophan sowie Mischungen hiervon. Besonders bevorzugte oxidative Behandlungsmittel enthalten Mischungen aus Arginin und Lysin oder mindestens einem Salz dieser Aminosäuren.

Bevorzugte erfindungsgemäße oxidative Behandlungsmittel enthalten die mindestens eine Aminosäure der Formel (VI) oder ein oder mehrere Salze hiervon in einer Gesamtmenge von 0,05 bis 3 Gew.-%, bevorzugt 0,1 bis 2 Gew.-%, besonders bevorzugt 0,2 bis 1,2 Gew.-%, jeweils umgerechnet auf die undissoziierte Säure und bezogen auf das Gewicht des Behandlungsmittels. Weitere besonders bevorzugte erfindungsgemäße Behandlungsmittel enthalten Mischungen aus Arginin und Lysin oder mindestens einem Salz dieser Aminosäuren in einer Gesamtmenge von 0,05 bis 3 Gew.-%, bevorzugt 0,1 bis 2 Gew.-%, besonders bevorzugt 0,2 bis 1,2 Gew.-%, jeweils umgerechnet auf die undissoziierte Säure und bezogen auf das Gewicht des Behandlungsmittels.

### Wasserstoffperoxid

Die Ausbildung der Farbstoffe in oxidativen Färbemitteln bzw. der Abbau des haareigenen Farbstoffs Melanin zur Blondierung erfolgt erst durch den Einfluss einer Peroxidverbindung als Oxidationsmittel. Üblicherweise wird hierfür Wasserstoffperoxid verwendet. Der Einsatz von Wasserstoffperoxid kann nur in Form einer wässrigen Lösung erfolgen.

Erfindungsgemäß bevorzugte oxidative Behandlungsmittel sind dadurch gekennzeichnet, dass sie 0,5 bis 13 Gew.-%, weiter bevorzugt 1 bis 7 Gew.-%, besonders bevorzugt 2 bis 6 Gew.-% und ganz besonders bevorzugt 3 bis 4,5 Gew.-% Wasserstoffperoxid (berechnet als 100 %-iges H₂O₂) enthalten, jeweils bezogen auf das Gesamtgewicht des erfindungsgemäßen oxidativen Behandlungsmittels.

Blondiermittel oder besonders stark aufhellende Färbemittel können darüber hinaus stark oxidierende Peroxidverbindungen, wie Kalium-, Natrium- und/oder Ammoniumpersulfat und/oder Natriumpercarbonat enthalten.

### Wasser

Die erfindungsgemäßen oxidativen Behandlungsmittel enthalten Wasser, und zwar bevorzugt in einer Menge von 20 bis 95 Gew.-%, bevorzugt 30 bis 90 Gew.-%, besonders bevorzugt 40 bis 85 Gew.-%, außerordentlich bevorzugt 50 bis 80 Gew.-%, jeweils bezogen auf das Gesamtgewicht des erfindungsgemäßen oxidativen Behandlungsmittels.

### pH-Wert

Erfindungsgemäße oxidative Behandlungsmittel weisen einen pH-Wert im Bereich von 2,5 - 6,5, bevorzugt im Bereich von 3 - 5,5, besonders bevorzugt im Bereich von 3,5 bis 5,0, auf, jeweils bei 20°C gemessen.

### Chelatbildner

Der erfindungsgemäße Effekt kann überraschend gesteigert werden, wenn das oxidative Behandlungsmittel mindestens einen Chelatbildner enthält. Unter einem Chelatbildner wird erfindungsgemäß eine Verbindung verstanden, die in wässriger Lösung mit Calciumionen oder Magnesiumionen Chelate bildet. Unter Chelaten werden erfindungsgemäß zyklische Verbindungen verstanden, bei denen Metallionen, weiterhin Gruppierungen mit einsamen Elektronenpaaren oder mit Elektronenlücken und weiterhin Wasserstoff an der Ringbildung beteiligt sind.

Erfindungsgemäß bevorzugte Chelatbildner weisen eine Molmasse von 100 g/mol bis 600 g/mol auf. Weitere erfindungsgemäß bevorzugte Chelatbildner sind ausgewählt aus Pyridin-2,6-dicarbonsäure (Dipicolinsäure), 1-Hydroxyethan-1,1-diphosphonsäure (HEDP, Etidronsäure) und deren Natrium-, Kalium- und Ammoniumsalzen, Dinatriumpyrophosphat, beta-Alanindiessigsäure, Ethylendiamintetraessigsäure (EDTA) und deren Natrium-, Kalium- und Ammoniumsalzen, Aminotrimethylenphosphonsäure, Diethylentriaminpentamethylenphosphonsäure, Dinatriumazacycloheptandiphosphonat, Dinatriumhydroxyethylaminobismethylphosphonat, Dinatriumhydroxyethyliminodiacetat, Hydroxyethylethylendiamintriessigsäure (HEDTA) und deren Natrium- oder Kaliumsalze, Pentakaliumtriphosphat, Pentanatriumtriphosphat, Pentanatriumaminotrimethylenphosphonat, Pentanatriumethylendiamintetramethylenphosphonat, Pentanatriumdiethylentriaminpentaacetat (Pentanatrium Pentetate), Diethylentriaminpentaessigsäure (Pentetic Acid), Phosphonobutantricarbonsäure, Kaliumethylendiamintetramethylenphosphonat (Kalium EDTMP), Natriumethylendiamintetramethylenphosphonat (Natrium EDTMP), Natriumdiethylentriaminpentamethylenphosphonat (Natrium DTPMP), Natriumhexametaphosphat, Tetrakaliumpyrophosphat, Tetranatriumpyrophosphat, Tetranatriumdicarboxymethylaspartat, Tetranatriumiminodisuccinat, Trinatriumdicarboxymethylalaninat, Trinatriumethylendiamindisuccinat (Trinatrium EDDS) und Trinatriumnitrilotriacetat sowie Mischungen dieser Chelatbildner.

Erfindungsgemäß besonders bevorzugte Chelatbildner sind ausgewählt aus Pyridin-2,6-dicarbonsäure (Dipicolinsäure), 1-Hydroxyethan-1,1-diphosphonsäure (HEDP, Etidronsäure) und deren Natrium-, Kalium- und Ammoniumsalzen, Dinatriumpyrophosphat, Ethylendiamintetraessigsäure (EDTA) und deren Kalium- und Ammoniumsalzen, Pentanatriumdiethylentriaminpentaacetat (Pentanatrium Pentetate), Tetranatriumpyrophosphat, Trinatriumethylendiamindisuccinat (Trinatrium EDDS) sowie Mischungen dieser Chelatbildner.

Erfindungsgemäß besonders bevorzugte oxidative Behandlungsmittel enthalten mindestens einen Chelatbildner in einer Gesamtmenge von 0,01 - 2,5 Gew.-%, bevorzugt in einer Gesamtmenge von 0,1 - 1,5 Gew.-%, besonders bevorzugt in einer Gesamtmenge von 0,2 - 0,6 Gew.-%, jeweils bezogen auf das Gewicht des oxidativen Behandlungsmittels.

Weiterhin wurde überraschend festgestellt, dass die verringerte Haarschädigungswirkung der erfindungsgemäßen und erfindungsgemäß bevorzugten oxidativen Behandlungsmittel weiter unterstützt werden kann, wenn mindestens eine Verbindung der allgemeinen Formel (III) enthalten ist. Erfindungsgemäß bevorzugte oxidative Behandlungsmittel enthalten daher
(a) mindestens eine Verbindung der allgemeinen Formel (III) 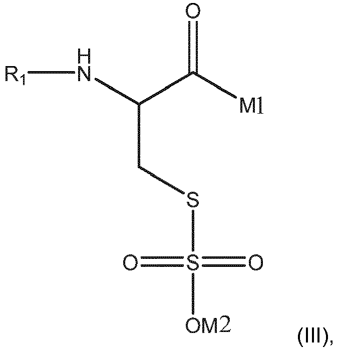 wobei
   R1 für ein Wasserstoffatom oder für ein Strukturelement der Formel (IV) steht 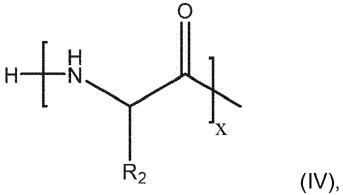 wobei
   x für eine ganze Zahl von 1 bis 100 steht,
   der Rest R2 in jedem der Strukturelemente der Formel (IV) jeweils unabhängig vom vorangegangenen Strukturelement der Formel (IV) gewählt werden kann,
   R2 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-yl-methyl-Gruppe oder eine (Sulfosulfanyl)methyl-Gruppe,
   M1 steht für die Gruppierung -OM2 oder für ein Strukturelement der Formel (V) 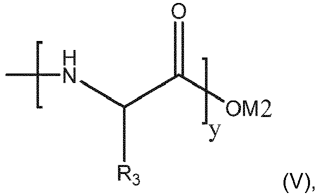
   wobei
   y für eine ganze Zahl von 1 bis 100 steht,
   der Rest R3 in jedem der Strukturelemente der Formel (V) jeweils unabhängig vom vorangegangenen Strukturelement der Formel (V) gewählt werden kann,
   R3 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   M2 steht für ein Wasserstoffatom, ein Äquivalent eines ein oder mehrwertigen Kations oder ein Ammoniumion (NH₄)⁺.

Bei dem wesentlichen Inhaltsstoff (a) der Formel (III) handelt es sich um das Bunte-Salz einer Aminosäure, eines Oligopeptids oder eines Peptids, das eine Verbindung der Formel (III) darstellt, 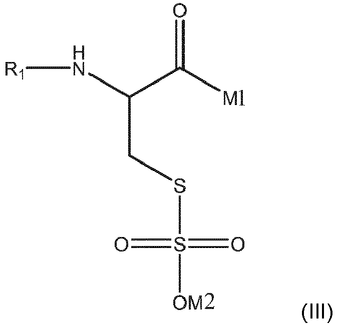 wobei
R1 für ein Wasserstoffatom oder für ein Strukturelement der Formel (IV) steht 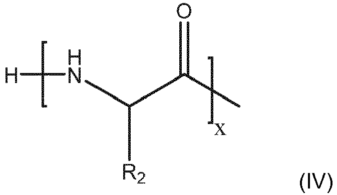 wobei
x für eine ganze Zahl von 1 bis 100 steht,
der Rest R2 in jedem der Strukturelemente der Formel (IV) jeweils unabhängig vom vorangegangenen Strukturelement der Formel (IV) gewählt werden kann,
R2 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (V) 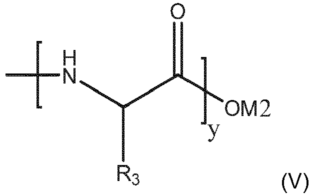 wobei
   y für eine ganze Zahl von 1 bis 100 steht
   der Rest R3 in jedem der Strukturelemente der Formel (V) jeweils unabhängig vom vorangegangenen Strukturelement der Formel (V) gewählt werden kann,
   R3 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   M2 steht für ein Wasserstoffatom, ein Äquivalent eines ein oder mehrwertigen Kations oder ein Ammoniumion (NH₄)⁺.

Der Rest R1 kann entweder für ein Wasserstoffatom oder für ein Strukturelement der Formel (IV) stehen

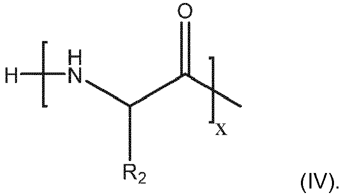

Das Strukturelement der Formel (IV) ist weiterhin gekennzeichnet durch den Wiederholungsindex x, wobei x für eine ganze Zahl von 1 bis 100 steht. Der Wiederholungsindex x gibt an, wie viele Strukturelemente der Formel (IV) in der Verbindung der Formel (III) enthalten sind.

Bevorzugt steht x für eine ganze Zahl von 1 bis 50, weiter bevorzugt steht x für eine ganze Zahl von 1 bis 20, und ganz besonders bevorzugt steht x für eine ganze Zahl von 1 bis 10.

Wenn x beispielweise für die Zahl 10 steht, beinhaltet die Verbindung der Formel (III) 10 Strukturelemente der Formel (IV).

Hierbei ist wesentlich, dass der Rest R2 in jedem der Strukturelemente der Formel (IV) jeweils unabhängig vom vorangegangenen Strukturelement der Formel (IV) gewählt werden kann. Enthalten die Verbindungen der Formel (III) beispielsweise 10 Struktureinheiten der Formel (IV), so können diese 10 Struktureinheiten gleich oder aber auch verschieden sein.

Der Rest R2 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, eine 1-Hydroxethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)-ethyl-Gruppe, eine 1H-Imidazol-4-ylmethyl-Gruppe, eine 1H-Indol-3-ylmethyl-Gruppe oder für eine (Sulfosulfanyl)methyl-Gruppe.

Bei dem Strukturelement der Formel (IV) handelt es sich somit um eine Aminosäure, die peptidisch über ihre Amino- und/oder ihre Säurefunktion innerhalb der Verbindung der Formel (III) verknüpft ist. Wenn es sich bei der Aminosäure um Cystein handelt, kann diese auch in Form eines Bunte-Salzes vorliegen.

Wenn der Rest R2 für ein Wasserstoffatom steht, so beruht das Strukturelement der Formel (IV) auf der Aminosäure Glycin.

Wenn der Rest R2 für eine Methylgruppe steht, so beruht das Strukturelement der Formel (IV) auf der Aminosäure Alanin.

Wenn der Rest R2 für eine Isopropylgruppe (d.h. eine Gruppe (H₃C)₂CH-) steht, so beruht das Strukturelement der Formel (IV) auf der Aminosäure Valin.

Wenn der Rest R2 für eine 2-Methylpropylgruppe (d.h. eine Gruppe (H₃C)₂CH-CH₂-), so beruht das Strukturelement der Formel (IV) auf der Aminosäure Leucin.

Wenn der Rest R2 für eine 1-Methyl-propylgruppe (d.h. eine Gruppe H3C-CH2-CH(CH3)-) steht, so beruht das Strukturelement der Formel (IV) auf der Aminosäure Isoleucin.

Wenn der Rest R2 für eine Benzylgruppe (d.h. eine Gruppe C₆H₅-CH₂-) steht, so beruht das Strukturelement der Formel (IV) auf der Aminosäure Phenylalanin.

Wenn der Rest R2 für eine 4-Hydroxybenzylgruppe (d.h. eine Gruppe 4-OH-C₆H₅-CH₂-) steht, so beruht das Strukturelement der Formel (IV) auf der Aminosäure Tyrosin.

Wenn der Rest R2 für eine Hydroxymethylgruppe (d.h. eine Gruppe HO-CH2-) steht, so beruht das Strukturelement der Formel (IV) auf der Aminosäure Serin.

Wenn der Rest R2 für eine 1-Hydroxyethylgruppe (d.h. eine Gruppe H3C-CH(OH)-) steht, so beruht das Strukturelement der Formel (IV) auf der Aminosäure Threonin.

Wenn der Rest R2 für eine 4-Aminobutylgruppe (d.h. eine Gruppe H2N-CH2-CH2-CH2-CH2-) steht, so beruht das Strukturelement der Formel (IV) auf der Aminosäure Lysin.

Wenn der Rest R2 für eine 3-Carbamimidamidopropyl-Gruppe (d.h. eine Gruppe H₂N-C(NH)-NH-CH₂-CH₂-CH₂-) steht, so beruht das Strukturelement der Formel (IV) auf der Aminosäure Arginin.

Wenn der Rest R2 für eine 2-Carboxyethyl-Gruppe (d.h. eine Gruppe HOOC-CH2-CH2-) steht, so beruht das Strukturelement der Formel (IV) auf der Aminosäure Glutaminsäure.

Wenn der Rest R2 für eine Carboxymethyl-Gruppe (d.h. eine Gruppe HOOC-CH2-) steht, so beruht das Strukturelement der Formel (IV) auf der Aminosäure Asparaginsäure.

Wenn der Rest R2 für eine 2-Carbamoylethyl-Gruppe (d.h. eine Gruppe H2N-C(O)-CH2-CH2-) steht, so beruht das Strukturelement der Formel (IV) auf der Aminosäure Glutamin.

Wenn der Rest R2 für eine Carbamoylmethyl-Gruppe (d.h. eine Gruppe H2N-C(O)-CH2-) steht, so beruht das Strukturelement der Formel (IV) auf der Aminosäure Asparagin.

Wenn der Rest R2 für eine Sulfanylmethyl-Gruppe (d.h. eine Gruppe HS-CH2-) steht, so beruht das Strukturelement der Formel (IV) auf der Aminosäure Cystein.

Wenn der Rest R2 für eine 2-(Methylsulfanyl)ethyl-Gruppe (d.h. eine Gruppe H3C-S-CH2-CH2-) steht, so beruht das Strukturelement der Formel (IV) auf der Aminosäure Methionin.

Wenn der Rest R2 für eine 1H-Imidazol-4-ylmethyl-Gruppe steht, so beruht das Strukturelement der Formel (IV) auf der Aminosäure Histidin.

Wenn der Rest R2 für eine 1H-Indol-3-ylmethyl-Gruppe steht, so beruht das Strukturelement der Formel (IV) auf der Aminosäure Tryptophan.

Schließlich kann der Rest R2 auch für eine (Sulfosulfanyl)methyl-Gruppe stehen, hierbei handelt es sich um eine Bunte-Salz-Struktur der Formel HO-S(O₂)-S-CH₂-.

Je nach pH-Wert des Behandlungsmittels kann die Bunte-Salz-Struktur der Formel HO-S(O₂)-S-CH₂- auch in ihrer deprotonierten Form vorliegen.

Innerhalb der Verbindung der Formel (III) steht M1 steht die Gruppierung -OM2 oder für ein Strukturelement der Formel (V)

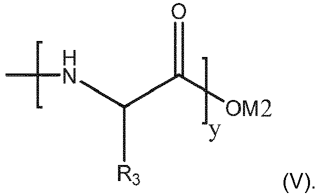

Das Strukturelement der Formel (V) ist gekennzeichnet durch den Wiederholungsindex y, wobei y für eine ganze Zahl von 1 bis 100 steht. Der Wiederholungsindex y gibt an, wie viele Strukturelemente der Formel (V) in der Verbindung der Formel (III) enthalten sind.

Bevorzugt steht y für eine ganze Zahl von 1 bis 50, weiter bevorzugt steht y für eine ganze Zahl von 1 bis 20, und ganz besonders bevorzugt steht y für eine ganze Zahl von 1 bis 10.

Wenn y beispielweise für die Zahl 10 steht, beinhaltet die Verbindung der Formel (III) 10 Strukturelemente der Formel (V).

Hierbei ist wesentlich, dass der Rest R3 in jedem der Strukturelemente der Formel (V) jeweils unabhängig vom vorangegangenen Strukturelement der Formel (V) gewählt werden kann. Enthalten die Verbindungen der Formel (III) beispielsweise 10 Struktureinheiten der Formel (V), so können diese 10 Struktureinheiten gleich oder aber auch verschieden sein.

Der Rest R3 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)-ethyl-Gruppe, eine 1H-Imidazol-4-ylmethyl-Gruppe, eine 1H-Indol-3-ylmethyl-Gruppe oder eine (Sulfosulfanyl)methyl-Gruppe.

Damit handelt es sich auch bei dem Strukturelement der Formel (V) um eine Aminosäure, die peptidisch über ihre Amino- und/oder ihre Säurefunktion innerhalb der Verbindung der Formel (III) verknüpft ist. Wenn es sich bei der Aminosäure um Cystein handelt, kann diese auch in Form eines Bunte-Salzes vorliegen.

Wenn der Rest R3 für ein Wasserstoffatom steht, so beruht das Strukturelement der Formel (V) auf der Aminosäure Glycin.

Wenn der Rest R3 für eine Methylgruppe steht, so beruht das Strukturelement der Formel (V) auf der Aminosäure Alanin.

Wenn der Rest R3 für eine Isopropylgruppe (d.h. eine Gruppe (H₃C)₂CH-) steht, so beruht das Strukturelement der Formel (V) auf der Aminosäure Valin.

Wenn der Rest R3 für eine 2-Methylpropylgruppe (d.h. eine Gruppe (H₃C)₂CH-CH₂-), so beruht das Strukturelement der Formel (V) auf der Aminosäure Leucin.

Wenn der Rest R3 für eine 1-Methyl-propylgruppe (d.h. eine Gruppe H3C-CH2-CH(CH3)-) steht, so beruht das Strukturelement der Formel (V) auf der Aminosäure Isoleucin.

Wenn der Rest R3 für eine Benzylgruppe (d.h. eine Gruppe C₆H₅-CH₂-) steht, so beruht das Strukturelement der Formel (V) auf der Aminosäure Phenylalanin.

Wenn der Rest R3 für eine 4-Hydroxybenzylgruppe (d.h. eine Gruppe 4OH-C₆H₅-CH₂-) steht, so beruht das Strukturelement der Formel (V) auf der Aminosäure Tyrosin.

Wenn der Rest R3 für eine Hydroxymethylgruppe (d.h. eine Gruppe HO-CH2-) steht, so beruht das Strukturelement der Formel (V) auf der Aminosäure Serin.

Wenn der Rest R3 für eine 1-Hydroxyethylgruppe (d.h. eine Gruppe H3C-CH(OH)-) steht, so beruht das Strukturelement der Formel (V) auf der Aminosäure Threonin.

Wenn der Rest R3 für eine 4-Aminobutylgruppe (d.h. eine Gruppe H2N-CH2-CH2-CH2-CH2-) steht, so beruht das Strukturelement der Formel (V) auf der Aminosäure Lysin.

Wenn der Rest R3 für eine 3-Carbamimidamidopropyl-Gruppe (d.h. eine Gruppe H₂N-C(NH)-NH-CH₂-CH₂-CH₂-) steht, so beruht das Strukturelement der Formel (V) auf der Aminosäure Arginin. Wenn der Rest R3 für eine 2-Carboxyethyl-Gruppe (d.h. eine Gruppe HOOC-CH2-CH2-) steht, so beruht das Strukturelement der Formel (V) auf der Aminosäure Glutaminsäure.

Wenn der Rest R3 für eine Carboxymethyl-Gruppe (d.h. eine Gruppe HOOC-CH2-) steht, so beruht das Strukturelement der Formel (V) auf der Aminosäure Asparaginsäure.

Wenn der Rest R3 für eine 2-Carbamoylethyl-Gruppe (d.h. eine Gruppe H2N-C(O)-CH2-CH2-) steht, so beruht das Strukturelement der Formel (V) auf der Aminosäure Glutamin.

Wenn der Rest R3 für eine Carbamoylmethyl-Gruppe (d.h. eine Gruppe H2N-C(O)-CH2-) steht, so beruht das Strukturelement der Formel (V) auf der Aminosäure Asparagin.

Wenn der Rest R3 für eine Sulfanylmethyl-Gruppe (d.h. eine Gruppe HS-CH2-) steht, so beruht das Strukturelement der Formel (V) auf der Aminosäure Cystein.

Wenn der Rest R3 für eine 2-(Methylsulfanyl)ethyl-Gruppe (d.h. eine Gruppe H3C-S-CH2-CH2-) steht, so beruht das Strukturelement der Formel (V) auf der Aminosäure Methionin.

Wenn der Rest R3 für eine 1H-Imidazol-4-ylmethyl-Gruppe steht, so beruht das Strukturelement der Formel (V) auf der Aminosäure Histidin.

Wenn der Rest R3 für eine 1H-Indol-3-ylmethyl-Gruppe steht, so beruht das Strukturelement der Formel (V) auf der Aminosäure Tryptophan.

Schließlich kann der Rest R3 auch für eine (Sulfosulfanyl)methyl-Gruppe stehen, hierbei handelt es sich um eine Bunte-Salz Struktur der Formel HO-S(O₂)-S-CH₂-.

Je nach pH-Wert des Behandlungsmittels kann auch hier die Bunte-Salz Struktur der Formel HO-S(O₂)-S-CH₂- auch in ihrer deprotonierten Form vorliegen.

Der Rest M2 steht für ein Wasserstoffatom, ein Äquivalent eines ein oder mehrwertigen Kations oder ein Ammoniumion (NH₄)⁺.

Als bevorzugte Äquivalente eines ein oder mehrwertigen Kations können insbesondere die Kationen von Natrium und Kalium (Na⁺ bzw. K⁺) oder auch Magnesium oder Calcium (1/2 Mg²⁺ oder ½ Ca²⁺) genannt werden.

Steht M2 für ein Wasserstoffatom, so handelt es sich bei der Gruppierung -OM2 um die Gruppierung -OH. Steht M2 für ein Natriumkation, so handelt es sich bei der Gruppierung -OM2 um die Gruppierung -ONa. Steht M2 für ein Kaliumkation, so handelt es sich bei der Gruppierung -OM2 um die Gruppierung -OK. Steht M2 für ein Ammoniumion, so handelt es sich bei der Gruppierung -OM2 um die Gruppierung -O(NH₄).

Die Gruppierung -OM2 ist stets einer Carbonylgruppe benachbart. In Summe liegt, wenn M2 für H, K, Na oder Ammonium steht, damit in der Verbindung der Formel (III) daher entweder in Form eine Säure in ihrer protonierten Form oder aber das Natrium, Kalium oder Ammoniumsalz dieser Säure vor.

Bei den erfindungsgemäßen Verbindungen der Formel (III) handelt es sich entweder um das Bunte-Salz der Aminosäure Cystein, um die Bunte-Salze von Oligopeptiden oder aber um die Bunte-Salze von Peptiden.

Wenn der Rest R1 für ein Wasserstoffatom steht und der Rest M1 für eine Gruppierung -OM2 steht, dann handelt es sich bei der Verbindung der Formel (III) um das Bunte-Salz der Aminosäure Cystein. In diesem Fall handelt es sich bei der Verbindung der Formel (III) um die Verbindung der Formel (lila), 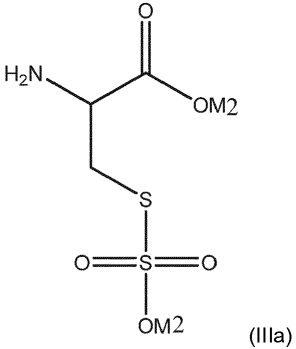 wobei M2 wieder wie zuvor beschrieben definiert.

Liegt die Verbindung der Formel (lila) in Form ihrer freien Säure vor, so handelt es sich um 2-Amino-3-(sulfosulfanyl)propansäure. Diese Substanz ist kommerziell erhältlich.

Es hat sich herausgestellt, dass der Einsatz der Verbindung der Formel (lila) in Behandlungsmittel bereits bei besonders geringen Einsatzmengen zu einer besonders effektiven Reduzierung der Haarschädigung führt, die auch nach wiederholten Haarwäschen noch vorhanden ist. Deshalb ist der Einsatz von Verbindungen der Formel (lila) in Behandlungsmitteln ganz besonders bevorzugt.

In einer ganz besonders bevorzugten Ausführungsform ist ein erfindungsgemäßes oxidatives Behandlungsmittel, dadurch gekennzeichnet, dass es mindestens eine Verbindung der Formel (III) enthält, wobei
R1 für ein Wasserstoffatom steht und
M1 für eine Gruppierung -OM2 steht.

Wenn eine Verbindung der Formel (lila) eingesetzt wird, so handelt es sich bevorzugt um den Einsatz dieser spezifischen Verbindung. Werden als Verbindungen der Formel (III) jedoch die Bunte-Salze von Oligopeptiden eingesetzt, so kann das erfindungsgemäße Behandlungsmittel auch mehrere Verbindungen der Formel (III) als Gemisch verschiedener Oligopeptide enthalten. Diese Oligopeptide werden durch ihr mittleres Molgewicht definiert. Das mittlere Molekulargewicht M_{w} des mindestens einen Oligopeptids der Formel (III) kann beispielsweise durch Gelpermeationschromatographie (GPC) mit Polystyrol als internem Standard gemäß DIN 55672-3, Version 8/2007, bestimmt werden.

Je nachdem, wie viele Strukturelemente der Formel (III) und/oder (IV) in der Verbindung der Formel (III) enthält sind, und je nach Art dieser Aminosäuren kann das Molgewicht der erfindungsgemäß verwendeten Verbindung der Formel (III) variieren. Es ist erfindungsgemäß besonders bevorzugt, wenn es sich bei der Verbindung der Formel (III) um ein Oligopeptid handelt, das ein Molekulargewicht M_{w} von 200 bis 2.000 Da, vorzugsweise von 250 bis 1.500 Da, bevorzugt von 300 bis 1.200 Da, insbesondere von 400 bis 800 Da aufweist.

Unter dem Begriff "Oligopeptid" im Rahmen der vorliegenden Erfindung werden Kondensationsprodukte von Aminosäuren verstanden, welche die oben genannten Molekulargewichte besitzen.

In einer ganz besonders bevorzugten Ausführungsform ist ein erfindungsgemäßes oxidatives Behandlungsmittel dadurch gekennzeichnet, dass es mindestens eine Verbindung der Formel (III) enthält, die ein Molekulargewicht M_{w} von 200 bis 2.000 Da (Dalton), vorzugsweise von 250 bis 1.500 Da, bevorzugt von 300 bis 1.200 Da, insbesondere von 400 bis 800 Da aufweist.

Wird im erfindungsgemäßen Behandlungsmittel eine Mischung von Oligomeren eingesetzt, so können diese Mischungen durch ihr mittleres Molekulargewicht definiert werden.

In diesem Fall ist ein bevorzugtes erfindungsgemäßes Behandlungsmittel dadurch gekennzeichnet, dass es mindestens eine Mischung von Verbindungen der Formel (III) enthält, die ein mittleres Molekulargewicht M_{w} von 200 bis 2.000 Da, vorzugsweise von 250 bis 1.500 Da, bevorzugt von 300 bis 1.200 Da, insbesondere von 400 bis 800 Da aufweist.

Weiterhin hat sich gezeigt, dass der Schutz- oder Repair-Effekt, den die Verbindungen der Formel (III) besitzen, auch von den Wiederholungsindices x und y abhängt. Wie zuvor beschrieben, ist es ganz besonders bevorzugt, wenn x für eine ganze Zahl von 1 bis 10 steht und y für eine ganze Zahl von 1 bis 10 steht.

In einer weiteren ganz besonders bevorzugten Ausführungsform ist ein erfindungsgemäßes oxidatives Behandlungsmittel dadurch gekennzeichnet, dass es mindestens eine Verbindung der Formel (III) enthält, wobei
R1 für ein Strukturelement der Formel (IV) steht, und
M1 für ein Strukturelement der Formel (V) steht, und
x für eine ganze Zahl von 1 bis 10 steht und
y für eine ganze Zahl von 1 bis 10 steht.

Neben dem Molekulargewicht der Verbindung der Formel (III) nimmt auch der Anteil der Bunte-Salz Einheiten, die in der Verbindung der Formel (III) enthalten sind, einen entscheidenden Einfluss auf die Wirksamkeit der Schutzwirkung oder "Repair-Wirkung" der Verbindungen.

Verbindungen mit mindestens einer Bunte-Salz-Einheit - wie sie beispielsweise in der Verbindung der Formel (IIIa) vorhanden ist-sind sehr effektiv, insbesondere wenn sie als monomere Verbindung eingesetzt werden. Oligopeptide mit mindestens einer Bunte-Salz Einheit sind besonders wirksam, wenn sie ein niedriges Molekulargewicht von bis zu 1200, insbesondere 800 Dalton haben.

Bei Einsatz von Oligopeptiden ist es jedoch von ganz besonderem Vorteil, wenn die Verbindung der Formel (III) mindestens zwei, bevorzugt mindestens drei Bunte-Salz Einheiten besitzt.

In einer weiteren ganz besonders bevorzugten Ausführungsform ist ein erfindungsgemäßes Behandlungsmittel dadurch gekennzeichnet, dass es mindestens eine Verbindung der Formel (III) enthält, wobei
R1 für ein Strukturelement der Formel (IV) steht, und
der Rest R2 in mindestens einem Strukturelement der Formel (IV) für eine (Sulfosulfanyl)methyl-Gruppe (d.h. eine Gruppe HO-S(O₂)-S-CH₂-) steht.

In einer weiteren ganz besonders bevorzugten Ausführungsform ist ein erfindungsgemäßes Behandlungsmittel dadurch gekennzeichnet, dass es mindestens eine Verbindung der Formel (III) enthält, wobei
R1 für ein Strukturelement der Formel (IV) steht, und
x für eine ganze Zahl von mindestens 3 steht und
der Rest R2 in mindestens 3 Strukturelementen der Formel (IV) für eine 2-Carboxyethyl-Gruppe (d.h. eine Gruppe HOOC-CH2-CH2-) steht.

In einer weiteren ganz besonders bevorzugten Ausführungsform ist ein erfindungsgemäßes Behandlungsmittel dadurch gekennzeichnet, dass es mindestens eine Verbindung der Formel (III) enthält, wobei
M1 für ein Strukturelement der Formel (V) steht, und
y für eine ganze Zahl von mindestens 3 steht und
der Rest R3 in mindestens 3 Strukturelementen der Formel (IV) für eine Gruppe (Glu) steht. Die mindestens eine Verbindung der Formel (III) ist - bezogen auf das Gesamtgewicht des erfindungsgemäß bevorzugten Behandlungsmittels in einer Gesamtmenge von 0,001 bis 10 Gew.-% enthalten. Überraschenderweise hat sich jedoch herausgestellt, dass die Verbindung(en) der Formel (III) bereits in geringen Einsatzkonzentrationen eine sehr gute Reduzierung der Haarschädigung bewirken können. Dies ist insbesondere dann von Vorteil, wenn die mindestens eine Verbindung der Formel (III) dem erfindungsgemäßen Behandlungsmittel als Additiv (beispielsweise in Form einer Pflegelösung oder Reparaturlösung) vor der Applikation auf das Haar hinzugefügt werden sollen. Aus diesem Grund ist es besonders vorteilhaft, wenn das erfindungsgemäß bevorzugte oxidative Behandlungsmittel eine oder mehrere Verbindungen der vorstehend aufgeführten Formel (III) in einer Gesamtmenge von 0,001 bis 2,5 Gew.-% weiter bevorzugt von 0,01 bis 1,0 Gew.-% und besonders bevorzugt von 0,02 bis 0,1 Gew.-% enthält, jeweils bezogen auf das Gewicht des erfindungsgemäßen oxidativen Behandlungsmittels.

In einer weiteren ganz besonders bevorzugten Ausführungsform ist ein erfindungsgemäßes oxidatives Behandlungsmittel dadurch gekennzeichnet, dass es eine oder mehrere Verbindungen der vorstehend aufgeführten Formel (III) in einer Gesamtmenge von 0,001 bis 2,5 Gew.-% weiter bevorzugt von 0,01 bis 1,0 Gew.-% und besonders bevorzugt von 0,02 bis 0,1 Gew.-% enthält, jeweils bezogen auf das Gewicht des erfindungsgemäßen Behandlungsmittels.

### Fettsäureamide

Der erfindungsgemäße Effekt kann überraschend gesteigert werden, wenn das oxidative Behandlungsmittel mindestens ein Fettsäureamid der Formel (AMID-I), wie nachstehend beschrieben, enthält. Erfindungsgemäß bevorzugte oxidative Behandlungsmittel enthalten mindestens ein Fettsäureamid der Formel (AMID-I), 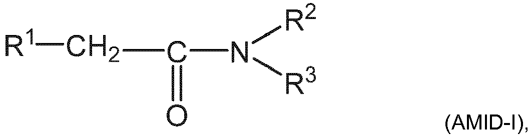 worin
R¹ ausgewählt ist aus linearen und verzweigten C4-C22-Alkylgruppen, die gesättigt oder ungesättigt sind, wobei die Alkyl- und Alkenylgruppen unsubstituiert sind oder mit Sauerstoffatomen, Stickstoffatomen, Hydroxylgruppen, Ethergruppen, Oxyalkylengruppen, Polyoxyalkylengruppen, Carbonsäuregruppen, Amingruppen, Amidgruppen, Halogen-enthaltenden Gruppen, Estergruppen, Siloxangruppen oder Polysiloxangruppen substituiert sein können,
und aus alkoxylierten Alkylgruppen der Formeln 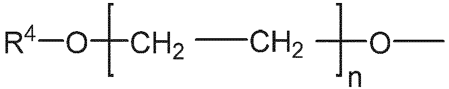 und 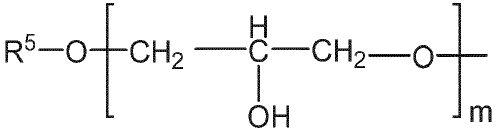 worin:
   R⁴ und R⁵, die gleich oder verschieden sein können, ausgewählt sind aus linearen und verzweigten C4-C22-Alkylgruppen, die gesättigt oder ungesättigt sind,
   n eine ganze Zahl im Bereich von 1 bis 10, bevorzugt im Bereich von 1 bis 4 ist und m eine ganze Zahl im Bereich von 1 bis 6 liegt und
   R² und R³, die gleich oder verschieden sein können, jeweils unabhängig voneinander ausgewählt sind aus H; linearen und verzweigten C1-C22-Alkylgruppen, die gesättigt oder ungesättigt sind, wobei die Alkyl- und Alkenylgruppen unsubstituiert sind oder mit Sauerstoffatomen, Stickstoffatomen, Hydroxylgruppen, Ethergruppen, Oxyalkylengruppen, insbesondere Oxyethylengruppen, Polyoxyalkylengruppen, insbesondere Polyoxyethylengruppen, Carbonsäuregruppen, Amingruppen, Alkylamingruppen, insbesondere C1-C3-Alkylamingruppen, Dialkylamingruppen, insbesondere Di-(C1-C3)-alkylamingruppen, substituierten und nicht substituierten 4,5-Dihydroimidazoliumgrupppen, Amidgruppen, Halogen enthaltenden Gruppen, Estergruppen, Siloxangruppen oder Polysiloxangruppen substituiert sind.

Bevorzugt ist mindestens ein Fettsäureamid der Formel (AMID-I), wie vorstehend dargestellt, in einer Gesamtmenge von 0,1 bis 10 Gew.-%, besonders bevorzugt 0,2 bis 5 Gew.-%, außerordentlich bevorzugt 0,4 - 2 Gew.-%, jeweils bezogen auf das Gewicht des oxidativen Behandlungsmittels, enthalten.

Besonders bevorzugte Fettsäureamide der Formel (AMID-I) sind solche, bei denen R¹ ausgewählt ist aus alkoxylierten Alkylgruppen der Formel 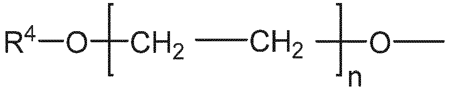 worin n eine ganze Zahl im Bereich von 1 bis 10, bevorzugt im Bereich von 1 bis 4 ist und R⁴ ausgewählt ist aus einer gesättigten linearen C4-C22-Alkylgruppe, R² Wasserstoff ist und R³ ausgewählt ist aus einer gesättigten linearen C1-C22-Alkylgruppe, die mit einer Hydroxylgruppe substituiert ist. Weitere besonders bevorzugte Fettsäureamide der Formel (AMID-I) sind solche, bei denen R¹ ausgewählt ist aus alkoxylierten Alkylgruppen der Formel 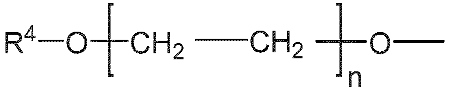 worin n 1 ist und R⁴ ausgewählt ist aus einer gesättigten linearen C11-C16-Alkylgruppe, R² Wasserstoff ist und R³ ausgewählt ist aus einer beta-Hydroxyethyl-Gruppe. Ein derartiges erfindungsgemäß besonders bevorzugtes Fettsäureamid ist zum Beispiel Trideceth-2 Carboxamide MEA.

Weitere besonders bevorzugte Fettsäureamide der Formel (AMID-I) sind solche, bei denen R¹ ausgewählt ist aus einer gesättigten linearen C11-C21-Alkylgruppe, R² Wasserstoff ist und R³ ausgewählt ist aus einer gesättigten linearen C1-C22-Alkylgruppe, die mit einer Amingruppe, einer C1-C3-Alkylamingruppe oder einer Di-(C1-C3)-alkylamingruppe substituiert ist. Weitere besonders bevorzugte Fettsäureamide der Formel (AMID-I) sind solche, bei denen R¹ ausgewählt ist aus einer gesättigten linearen C11-C21-Alkylgruppe, R² Wasserstoff ist und R³ ausgewählt ist aus einer gamma-Dimethylamin-n-propyl-Gruppe. Derartige erfindungsgemäß besonders bevorzugte Fettsäureamide sind zum Beispiel Stearamidopropyldimethylamin und Behenamidopropyldimethylamin.

Weitere besonders bevorzugte Fettsäureamide der Formel (AMID-I) sind solche, bei denen R¹ ausgewählt ist aus einer gesättigten linearen C11-C21-Alkylgruppe, R² Wasserstoff ist und R³ ausgewählt ist aus einer gesättigten linearen C1-C3-Alkylgruppe, die in omega-Position mit einer 4,5-Dihydroimidazoliumgrupppe substituiert ist, wobei letztere in 2-Position mit einer C8-C22-Alkylgruppe und in 1-Position mit einer Methylgruppe substituiert ist. Weitere besonders bevorzugte Fettsäureamide der Formel (AMID-I) sind solche, bei denen R¹ ausgewählt ist aus einer gesättigten linearen C11-C21-Alkylgruppe, R² Wasserstoff ist und R³ ausgewählt ist aus einer Ethylgruppe, die in omega-Position mit einer 4,5-Dihydroimidazoliumgrupppe substituiert ist, wobei letztere in 2-Position mit einer C8-C22-Alkylgruppe und in 1-Position mit einer Methylgruppe substituiert ist. Derartige erfindungsgemäß besonders bevorzugte Fettsäureamide sind zum Beispiel 1-(Docosanamidoethyl)-2-heneicosyl-1-methyl-4,5-dihydroimidazoliummethylsulfat, das die INCI-Bezeichnung "Quaternium-91" trägt, und 1-(Cocoylamidoethyl)-2-cocoyl-1-methyl-4,5-dihydroimidazoliummethylsulfat, das die INCI-Bezeichnung "Quaternium-87" trägt und die CAS-Nummer 92201-88-2 hat. Erfindungsgemäß besonders bevorzugte oxidative Behandlungsmittel enthalten, jeweils bezogen auf ihr Gewicht, in einer Gesamtmenge von 0,1 bis 10 Gew.-%, besonders bevorzugt 0,2 bis 5 Gew.- %, außerordentlich bevorzugt 0,4 - 2 Gew.-%, mindestens eines der Fettsäureamide der Formel (AMID-I), darunter mindestens eine Verbindung, die ausgewählt ist aus Trideceth-2 Carboxamide MEA, Stearamidopropyldimethylamin, Behenamidopropyldimethylamin, Quaternium-87 und Quaternium-91.

Die erfindungsgemäßen oxidativen Behandlungsmittel können direkt und ohne eine weitere Zusammensetzung zuzugeben auf die zu behandelnden Keratinfasern, insbesondere auf das Humanhaar, aufgetragen werden. Diese Applikationsart ist insbesondere dazu geeignet, eine leichte Aufhellung oder Blondierung der Keratinfasern zu erzielen und/oder den Aufhelleffekt einer vorhergehenden Blondierung länger zu konservieren. Um allerdings eine stärkere Aufhell- oder Blondierwirkung zu erzielen, muss das erfindungsgemäße oxidative Behandlungsmittel durch Zugabe eines Alkalisierungsmittels auf einen pH-Wert im Bereich 6 von 6,5 bis 12 (gemessen bei 20°C) eingestellt werden. Da alkalische Wasserstoffperoxidlösungen nicht lagerstabil sind, erfolgt die Zugabe des Alkalisierungsmittels erst kurz vor der Applikation auf die Keratinfasern. Dieses Alkalisierungsmittel, im Folgenden als Zusammensetzung (B) bezeichnet, kann fest, insbesondere pulverförmig sein, aber auch flüssig (0 bis 500 mPas bei 20°C), mittelviskos (> 400 bis 4000 mPas bei 20°C) oder creme- oder pastenförmig (> 4000 bis 4.000.000 mPas bei 20°C). Sofern die Zusammensetzung (B) pulverförmig ist, ist es erfindungsgemäß bevorzugt, dass sie mindestens ein Persulfatsalz und/oder mindestens ein Percarbonatsalz enthält.

Die Alkalisierungsmittel-haltige Zusammensetzung (B) kann sowohl wasserfrei als auch wasserhaltig sein. Wenn die Mischung aus erfindungsgemäßem oxidativen Behandlungsmittel und Alkalisierungsmittel-haltiger Zusammensetzung (B) zum Aufhellen oder Blondieren von Keratinfasern, insbesondere von Humanhaar, bestimmt ist, enthält die Zusammensetzung (B) üblicherweise keine Oxidationsfarbstoffvorprodukte. Es ist aber möglich, dass sie bestimmte direktziehende Farbstoffe enthält, die einen unerwünschten Rot- oder Orangestich der Melaninabbauprodukte kompensieren. Diese Technologie ist zum Beispiel in WO2002074270A1 oder in EP 2306963A1 offenbart.

### Alkalisierungsmittel für Zusammensetzung (B)

Die zur Kombination mit dem erfindungsgemäßen oxidativen Behandlungsmittel geeigneten Zusammensetzungen (B) enthalten mindestens ein Alkalisierungsmittel, ausgewählt aus Ammoniumhydroxid, Monoethanolamin und Natriumsilikaten sowie Mischungen hiervon.

Zur Erzielung der gewünschten permanenten Färbung oder Aufhellung der keratinischen Fasern muss die Zusammensetzung (B), die mit dem erfindungsgemäßen oxidativen Behandlungsmittel zum anwendungsbereiten Färbe- oder Aufhellmittel vermischt wird, einen pH-Wert im Bereich von 6,5 bis 11,0, bevorzugt 8 bis 10,5, besonders bevorzugt 8,5 bis 10, aufweisen, jeweils gemessen bei 20°C. Weiterhin ist es für das Färbe- oder Aufhellergebnis wichtig, dass das anwendungsbereite Färbe- oder Aufhellmittel, das durch Vermischen des erfindungsgemäßen oxidativen Behandlungsmittels mit der Zusammensetzung (B) erhalten wird, einen pH-Wert im Bereich von 6,5 bis 11,0, bevorzugt 8 bis 10,5, besonders bevorzugt 8,5 bis 9,5, aufweist, jeweils gemessen bei 20°C. Bei diesen pH-Werten öffnet sich die äußere Keratinfaserschicht optimal zur Aufnahme der Oxidationsfarbstoffvorprodukte und/oder entfaltet sich die oxidative Wirkung des Wasserstoffperoxids und ggf. weiterer Peroxidverbindungen optimal.

Bevorzugt wird Ammoniak in Form seiner wässrigen Lösung eingesetzt. Bei entsprechenden wässrigen Ammoniak-Lösungen kann es sich um 10 bis 35 prozentige Lösungen handeln (berechnet in Gew.-%, 100 g wässrige Ammoniaklösung enthalten dementsprechend 10 bis 35 g Ammoniak).

Bevorzugt wird Ammoniak in Form einer 20 bis 30 Gew.-%igen Lösung, besonders bevorzugt in Form einer 25 Gew.-%igen Lösung eingesetzt.

In einer besonders bevorzugten Ausführungsform ist die erfindungsgemäß verwendete Zusammensetzung (B) dadurch gekennzeichnet, dass sie Ammoniumhydroxid in einer Menge von 0,20 bis 18,0 Gew.-%, bevorzugt von 1,0 bis 15 Gew.-%, weiter bevorzugt von 2,0 bis 12,0 Gew.-% und besonders bevorzugt von 3,0 bis 9 Gew.-% - bezogen auf das Gewicht der erfindungsgemäß verwendeten Zusammensetzung (B) - enthält.

Zusätzlich zu bzw. anstelle von Ammoniumhydroxid enthalten bevorzugte erfindungsgemäß verwendete Zusammensetzungen (B) Monoethanolamin.

Zur Erzielung einer maximalen Geruchsabdeckung und zur Optimierung der Echtheitseigenschaften ist Monoethanolamin in einer Gesamtmenge von 0,2 - 9,0 Gew.-%, bevorzugt von 1,0-7 Gew.-%, weiter bevorzugt von 2,0 bis 6,0 Gew.-% und besonders bevorzugt von 3,0 bis 5,5 Gew.-% - bezogen auf das Gewicht der erfindungsgemäß verwendeten Zusammensetzung (B) - enthalten.

Bei Natriumsilikaten im Sinne der vorliegenden Erfindung handelt es sich um chemische Verbindungen, die sich aus Natriumoxid und Siliciumdioxid zusammensetzen und die in verschiedenen molaren Verhältnissen (Monosilikat, Metasilikat und Polysilikat) vorkommen können. Ein Beispiel für ein Natriumsilikat ist das Natriumsalz der Orthokieselsäure mit der Summenformel Na₄SiO₄, das auch als Natriumorthosilikat bezeichnet wird.

Weitere Beispiele für geeignete Natriumsilikate sind das Dinatriummetasilikat bzw. Natriummetasilikat mit der Summenformel Na₂SiO₃, das Dinatriumdisilikat mit der Summenformel Na₂Si₂O₅ oder das Dinatriumtrisilikat mit der Summenformel Na₂Si₃O₇.

Silikate in amorpher Form können durch das Zusammenschmelzen von Siliciumdioxid und Alkalioxid in molaren Verhältnissen zwischen 1:1 und 4:1 hergestellt werden. Die so erhaltenen Feststoffe werden bei etwa 150 °C und 5 bar Dampfdruck gelöst, um eine Lösung der Natriumsilikate in Wasser zu erhalten, bei diesen entsprechenden Lösungen handelt es sich um Alkaliwassergläser.

Als Alkaliwassergläser werden aus einer Schmelze erstarrte, glasartige (amorphe) Natriumsilikate oder ihre wässrigen Lösungen bezeichnet. Diese nennt man auch Natronwasserglas. Auch Natronwassergläser sind innerhalb dieser Erfindung von der Definition der Natriumsilikate umfasst.

Die molare Zusammensetzung bei Wassergläsern beträgt üblicherweise 2 bis 4 mol SiO₂ auf 1 mol Alkalioxid (Na₂O).

Ein Beispiel für ein bevorzugtes Natriumsilikat ist Natronwasserglas, das in Form seiner wässrigen Lösung vorliegt, einen Na₂O-Gehalt von 7,5 bis 8,8 Gew.-% und einen SiO2 Gehalt von 25,0 bis 28,5 Gew.-% besitzt und das die CAS-Nr. 1344-09-5 (Chemical Abstracts Number) hat.

Weitere erfindungsgemäß bevorzugt verwendete Zusammensetzungen (B) enthalten mindestens ein Natriumsilikat in einer Gesamtmenge von 0,1 bis 9 Gew.-%, bevorzugt 0,2 bis 8 Gew.-%, besonders bevorzugt 1 bis 7,5 Gew.-%, jeweils bezogen auf das Gewicht der erfindungsgemäß verwendeten Zusammensetzung (B).

Weiterhin können andere Alkalisierungsmittel, wie Kaliumhydroxid (KOH) und Natriumhydroxid (NaOH) enthalten sein, meist in einer Gesamtmenge von 0,05 bis 1,5 Gew.-%, bevorzugt 0,1 bis 0,6 Gew.-%, jeweils bezogen auf das Gewicht des der erfindungsgemäß verwendeten Zusammensetzung (B).

Wenn die Mischung aus erfindungsgemäßem oxidativen Behandlungsmittel und Alkalisierungsmittel-haltiger Zusammensetzung (B) zum oxidativen Färben von Keratinfasern, insbesondere von Humanhaar, bestimmt ist, enthält die Zusammensetzung (B) zur Ausbildung der Farbstoffe mindestens ein Oxidationsfarbstoffvorprodukt.

Unter die Oxidationsfarbstoffvorprodukte fallen Oxidationsfarbstoffvorprodukte vom Entwickler-Typ und vom Kuppler-Typ. Besonders geeignete Oxidationsfarbstoffvorprodukte vom Entwickler-Typ sind dabei ausgewählt aus mindestens einer Verbindung aus der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophen-oxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind ausgewählt aus der Gruppe 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis-(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

In einer bevorzugten Ausführungsform enthalten die erfindungsgemäß verwendeten Zusammensetzungen (B) ein oder mehrere Oxidationsfarbstoffvorprodukte in einer Gesamtmenge von 0,01 bis 30,0 Gew.-%, bevorzugt von 0,1 bis 15 Gew.-%, weiter bevorzugt von 0,6 bis 3,1 Gew.-% und ganz besonders bevorzugt von 1,2 bis 2,2 Gew.-%, bezogen auf das Gewicht der erfindungsgemäß verwendeten Zusammensetzung (B).

In einer weiteren bevorzugten Ausführungsform enthalten die erfindungsgemäßen oxidativen Behandlungsmittel oder die erfindungsgemäß verwendeten Zusammensetzungen (B) zusätzlich mindestens einen weiteren direktziehenden Farbstoff. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen. Die direktziehenden Farbstoffe sind jeweils bevorzugt in einer Gesamtmenge von 0,001 bis 2 Gew.-%, bezogen auf das Gewicht der erfindungsgemäß verwendeten Zusammensetzung (B) oder bezogen auf das Gewicht des erfindungsgemäßen oxidativen Behandlungsmittels, enthalten. Direktziehende Farbstoffe dienen in oxidativen Färbemitteln zur Nuancierung des erzielten Farbtons, in oxidativen Blondiermitteln zum Ausgleich unerwünschter Rottöne, die beim Abbau des haareigenen Melanins entstehen können.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterozyklus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Als weiteren optionalen Inhaltsstoff enthalten erfindungsgemäß bevorzugte oxidative Behandlungsmittel und/oder erfindungsgemäß bevorzugt verwendete Zusammensetzungen (B) mindestens ein permanent kationisches Polymer B.

Bevorzugt enthält das permanent kationische Polymer neben mindestens einem permanent kationisch geladenen Monomer-Typ auch mindestens einen permanent anionisch geladenen Monomer-Typ, wobei die kationischen Monomere im molaren Überschuss zu den anionischen Monomeren vorliegen, so dass das mindestens eine zweite erfindungsgemäße Polymer eine kationische Nettoladung aufweist. Derartige erfindungsgemäß bevorzugte Polymere werden auch als amphotere oder zwitterionische Polymere bezeichnet.

In einer ersten bevorzugten Ausführungsform enthalten erfindungsgemäß bevorzugte oxidative Behandlungsmittel und/oder erfindungsgemäß bevorzugt verwendete Zusammensetzungen (B) mindestens ein permanent kationisches Polymer, das ausgewählt ist aus
- kationischen Polymeren, die aufgebaut sind aus Monomeren mit quartären Ammoniumgruppen der allgemeinen Formel (IIa),

   R³-CH=CR⁴-CO-Z-(CₙH₂ₙ)-N⁽⁺⁾R⁵R⁶R⁷ A⁽⁻⁾ (IIa),

   in der R³ und R⁴ unabhängig voneinander für Wasserstoff oder eine Methylgruppe stehen, R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt,
   bevorzugt ausgewählt aus kationischen Polymeren, die aufgebaut sind aus Acrylamidopropyltrimethylammoniumchlorid,
   besonders bevorzugt ausgewählt aus amphoteren Polymeren mit kationischer Nettoladung, die durch Polymerisation aufgebaut sind aus
   a) kationischen Monomeren mit quartären Ammoniumgruppen der allgemeinen Formel (IIa),

      R³-CH=CR⁴-CO-Z-(CₙH₂ₙ)-N⁽⁺⁾R⁵R⁶R⁷ A⁽⁻⁾ (IIa),

      in der R³ und R⁴ unabhängig voneinander für Wasserstoff oder eine Methylgruppe stehen, R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt, und
   b) mindestens einer ungesättigten Carbonsäure, ausgewählt aus Acrylsäure, Methacrylsäure und Crotonsäure sowie aus Mischungen dieser Säuren, wobei die mindestens eine ungesättigte Carbonsäure in Form ihrer Salze vorliegen kann,
   wobei im Polymer die kationischen Monomere im molaren Überschuss zu den anionischen Monomeren vorliegen; außerordentlich bevorzugt ausgewählt aus amphoteren Polymeren mit kationischer Nettoladung, die den mindestens einen Monomer-Typ der allgemeinen Formel (IIa) und den mindestens einen Monomer-Typ der ungesättigten Carbonsäure, ausgewählt aus Acrylsäure, Methacrylsäure und Crotonsäure sowie Mischungen hiervon, in einem Molverhältnis von 60:40 bis 95:5, bevorzugt von 75:25 bis 90:10 zueinander enthalten,
   außerordentlichst bevorzugt ausgewählt aus amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen;
- 2-[2-Hydroxy-3-(trimethylammonio)propoxy]ethylcelluloseetherchlorid, das beispielsweise unter der INCI-Bezeichnung Polyquaternium-10 erhältlich ist,
- Terpolymeren aus Acrylsäure, Diallyldimethylammoniumchlorid und Acrylamid, wie sie beispielsweise unter der INCI-Bezeichnung Polyquaternium-39 erhältlich sind,
- Homopolymeren des N,N,N-Trimethyl-2-[(methyl-1-oxo-2-propenyl)oxy]ethanaminiumchlorid, wie sie beispielsweise unter der INCI-Bezeichnung Polyquaternium-37 erhältlich sind,
- Copolymeren aus Diallyldimethylammoniumchlorid und Acrylsäure, wie sie beispielsweise unter der INCI-Bezeichnung Polyquaternium-22 erhältlich sind,
- Hydroxyethylcellulose-Dimethyldiallylammoniumchlorid-Copolymeren, wie sie beispielsweise unter der INCI-Bezeichnung Polyquaternium-4 erhältlich sind,
- Copolymeren aus Acrylamid und beta-Methacrylyloxyethyltrimethylammoniummethosulfat, wie sie beispielsweise unter der INCI-Bezeichnung Polyquaternium-5 erhältlich sind,
- Homopolymeren des N,N-Dimethyl-N-2-Propenyl-2-Propen-1-aminiumchlorid, wie sie beispielsweise unter der INCI-Bezeichnung Polyquaternium-6 erhältlich sind,
- Copolymeren aus Diallyldimethylammoniumchlorid und Acrylamid, wie sie beispielsweise unter der INCI-Bezeichnung Polyquaternium-7 erhältlich sind,
- Copolymeren aus Vinylpyrrolidon und Dimethylaminoethylmethacrylatdiethylsulfat, wie sie beispielsweise unter der INCI-Bezeichnung Polyquaternium-11 erhältlich sind,
- sowie Mischungen der vorgenannten Polymere.

Erfindungsgemäß außerordentlich bevorzugte permanente kationische Polymere sind ausgewählt aus 2-[2-Hydroxy-3-(trimethylammonio)propoxy]ethylcelluloseetherchlorid, amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen und Terpolymeren aus Acrylsäure, Diallyldimethylammoniumchlorid und Acrylamid, sowie Zweier- und Dreier-Mischungen dieser Polymere.

Besonders bevorzugte Polymer B-Mischungen enthalten 2-[2-Hydroxy-3-(trimethylammonio)propoxy]ethylcelluloseetherchlorid und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht.

Weitere besonders bevorzugte Polymer B-Mischungen enthalten 2-[2-Hydroxy-3-(trimethylammonio)propoxy]ethylcelluloseetherchlorid, mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und mindestens ein Terpolymer aus Acrylsäure, Diallyldimethylammoniumchlorid und Acrylamid.

Erfindungsgemäß ebenfalls außerordentlich bevorzugte permanent-kationische Polymere B sind ausgewählt aus Polyquaternium-10, amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen, und Polyquaternium-39, sowie Zweier- und Dreier-Mischungen dieser Polymere.

Weitere besonders bevorzugte Polymer B-Mischungen enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht.

Weitere besonders bevorzugte Polymer B-Mischungen enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und Polyquaternium-39.

Bevorzugte erfindungsgemäße oxidative Behandlungsmittel enthalten das mindestens eine permanent kationische Polymer B in einer Gesamtmenge von 0,05 bis 1,5 Gew.-%, bevorzugt 0,1 bis 1,0 Gew.-%, besonders bevorzugt 0,2 bis 0,8 Gew.-%, jeweils bezogen auf das Gewicht des erfindungsgemäßen oxidativen Behandlungsmittels.

Bevorzugte erfindungsgemäß verwendete Zusammensetzungen (B) enthalten das mindestens eine permanent kationische Polymer B in einer Gesamtmenge von 0,05 bis 1,5 Gew.-%, bevorzugt 0,1 bis 1,0 Gew.-%, besonders bevorzugt 0,2 bis 0,8 Gew.-%, jeweils bezogen auf das Gewicht der erfindungsgemäß verwendeten Zusammensetzung (B).

Weitere erfindungsgemäß bevorzugte Behandlungsmittel und/oder erfindungsgemäß bevorzugt verwendete Zusammensetzungen (B) enthalten mindestens ein permanent kationisches Polymer B, ausgewählt aus
- kationischen Polymeren, die aufgebaut sind aus Monomeren mit quartären Ammoniumgruppen der allgemeinen Formel (IIa),

   R³-CH=CR⁴-CO-Z-(CₙH₂ₙ)-N⁽⁺⁾R⁵R⁶R⁷ A⁽⁻⁾ (IIa),

   in der R³ und R⁴ unabhängig voneinander für Wasserstoff oder eine Methylgruppe stehen, R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt,
   bevorzugt ausgewählt aus kationischen Polymeren, die aufgebaut sind aus Acrylamidopropyltrimethylammoniumchlorid,
   besonders bevorzugt ausgewählt aus amphoteren Polymeren mit kationischer Nettoladung, die durch Polymerisation aufgebaut sind aus
   c) kationischen Monomeren mit quartären Ammoniumgruppen der allgemeinen Formel (IIa),

      R³-CH=CR⁴-CO-Z-(CₙH₂ₙ)-N⁽⁺⁾R⁵R⁶R⁷ A⁽⁻⁾ (IIa),

      in der R³ und R⁴ unabhängig voneinander für Wasserstoff oder eine Methylgruppe stehen, R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt, und
   d) mindestens einer ungesättigten Carbonsäure, ausgewählt aus Acrylsäure, Methacrylsäure und Crotonsäure sowie aus Mischungen dieser Säuren, wobei die mindestens eine ungesättigte Carbonsäure in Form ihrer Salze vorliegen kann,
   wobei im Polymer die Gesamtheit aller kationischen Monomere im molaren Überschuss zur Gesamtheit aller anionischen Monomere vorliegt;
   außerordentlich bevorzugt ausgewählt aus amphoteren Polymeren mit kationischer Nettoladung, die den mindestens einen Monomer-Typ der allgemeinen Formel (IIa) und den mindestens einen Monomer-Typ der ungesättigten Carbonsäure, ausgewählt aus Acrylsäure, Methacrylsäure und Crotonsäure sowie Mischungen hiervon, in einem Molverhältnis von 60:40 bis 95:5, bevorzugt von 75:25 bis 90:10 zueinander enthalten,
   außerordentlichst bevorzugt ausgewählt aus amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen;
- 2-[2-Hydroxy-3-(trimethylammonio)propoxy]ethylcelluloseetherchlorid, das beispielsweise unter der INCI-Bezeichnung Polyquaternium-10 erhältlich ist,
- Terpolymeren aus Acrylsäure, Diallyldimethylammoniumchlorid und Acrylamid, wie sie beispielsweise unter der INCI-Bezeichnung Polyquaternium-39 erhältlich sind,
- Homopolymeren des N,N,N-Trimethyl-2-[(methyl-1-oxo-2-propenyl)oxy]ethanaminiumchlorid, wie sie beispielsweise unter der INCI-Bezeichnung Polyquaternium-37 erhältlich sind,
- Copolymeren aus Diallyldimethylammoniumchlorid und Acrylsäure, wie sie beispielsweise unter der INCI-Bezeichnung Polyquaternium-22 erhältlich sind,
- Hydroxyethylcellulose-Dimethyldiallylammoniumchlorid-Copolymeren, wie sie beispielsweise unter der INCI-Bezeichnung Polyquaternium-4 erhältlich sind,
- Copolymeren aus Acrylamid und beta-Methacrylyloxyethyltrimethylammoniummethosulfat, wie sie beispielsweise unter der INCI-Bezeichnung Polyquaternium-5 erhältlich sind,
- Homopolymeren des N,N-Dimethyl-N-2-Propenyl-2-Propen-1-aminiumchlorid, wie sie beispielsweise unter der INCI-Bezeichnung Polyquaternium-6 erhältlich sind,
- Copolymeren aus Diallyldimethylammoniumchlorid und Acrylamid, wie sie beispielsweise unter der INCI-Bezeichnung Polyquaternium-7 erhältlich sind, sowie
- Copolymeren aus Vinylpyrrolidon und Dimethylaminoethylmethacrylatdiethylsulfat, wie sie beispielsweise unter der INCI-Bezeichnung Polyquaternium-11 erhältlich sind,
in einer Gesamtmenge von 0,05 bis 1,5 Gew.-%, bevorzugt 0,1 bis 1,0 Gew.-%, besonders bevorzugt 0,2 bis 0,8 Gew.-%, jeweils bezogen auf das Gewicht des erfindungsgemäßen oxidativen Behandlungsmittels bzw. das Gewicht der erfindungsgemäß verwendeten Zusammensetzung (B).

Weitere erfindungsgemäß bevorzugte oxidative Behandlungsmittel und/oder erfindungsgemäß bevorzugt verwendete Zusammensetzungen (B).enthalten 2-[2-Hydroxy-3-(trimethylammonio)propoxy]ethylcelluloseetherchlorid und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, in einer Gesamtmenge von 0,05 bis 1,5 Gew.-%, bevorzugt 0,1 bis 1,0 Gew.-%, besonders bevorzugt 0,2 bis 0,8 Gew.-%, jeweils bezogen auf das Gewicht des erfindungsgemäßen oxidativen Behandlungsmittels bzw. das Gewicht der erfindungsgemäß verwendeten Zusammensetzung (B).

Weitere erfindungsgemäß bevorzugte oxidative Behandlungsmittel und/oder erfindungsgemäß bevorzugt verwendete Zusammensetzungen (B) enthalten 2-[2-Hydroxy-3-(trimethylammonio)propoxy]ethylcelluloseetherchlorid, mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und mindestens ein Terpolymer aus Acrylsäure, Diallyldimethylammoniumchlorid und Acrylamid, in einer Gesamtmenge von 0,05 bis 1,5 Gew.-%, bevorzugt 0,1 bis 1,0 Gew.-%, besonders bevorzugt 0,2 bis 0,8 Gew.-%, jeweils bezogen auf das Gewicht des erfindungsgemäßen oxidativen Behandlungsmittels bzw. das Gewicht der erfindungsgemäß verwendeten Zusammensetzung (B).

Weitere besonders bevorzugte oxidative Behandlungsmittel und/oder erfindungsgemäß bevorzugt verwendete Zusammensetzungen (B) enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, in einer Gesamtmenge von 0,05 bis 1,5 Gew.-%, bevorzugt 0,1 bis 1,0 Gew.-%, besonders bevorzugt 0,2 bis 0,8 Gew.-%, jeweils bezogen auf das Gewicht des erfindungsgemäßen oxidativen Behandlungsmittels bzw. das Gewicht der erfindungsgemäß verwendeten Zusammensetzung (B).

Weitere besonders bevorzugte oxidative Behandlungsmittel und/oder erfindungsgemäß bevorzugt verwendete Zusammensetzungen (B) enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und Polyquaternium-39 in einer Gesamtmenge von 0,05 bis 1,5 Gew.-%, bevorzugt 0,1 bis 1,0 Gew.-%, besonders bevorzugt 0,2 bis 0,8 Gew.-%, jeweils bezogen auf das Gewicht des erfindungsgemäßen oxidativen Behandlungsmittels bzw. das Gewicht der erfindungsgemäß verwendeten Zusammensetzung (B).

Ein weiterer Gegenstand der vorliegenden Erfindung ist ein Verfahren zur oxidativen Färbung und/oder Aufhellung keratinischer Fasern, insbesondere von Humanhaar, bei dem ein oxidatives Behandlungsmittel auf die keratinischen Fasern, insbesondere auf das Humanhaar, aufgetragen und ggf. nach einer Einwirkzeit von 0,1 bis 60 Minuten, bevorzugt 1 bis 50 Minuten, besonders bevorzugt 10 bis 45 Minuten, außerordentlich bevorzugt 30 bis 45 Minuten, wieder ausgespült wird, wobei dieses Behandlungsmittel
b) mindestens eine Dicarbonsäure mit 2 bis 10 Kohlenstoffatomen und/oder mindestens ein Salz dieser Säure(n),
c) mindestens eine Aminosäure,
e) Wasserstoffperoxid und
f) Wasser
enthält, wobei das oxidative Behandlungsmittel einen pH-Wert im Bereich von 2,5 - 6,5, bevorzugt im Bereich von 3 - 5,5, besonders bevorzugt im Bereich von 3,5 bis 5,0, aufweist, jeweils bei 20°C gemessen.

Für das erfindungsgemäße Verfahren zur oxidativen Färbung und/oder Aufhellung keratinischer Fasern, insbesondere von Humanhaar, und seine bevorzugten Ausführungsformen gilt mutatis mutandis das zu den erfindungsgemäßen und erfindungsgemäß bevorzugten oxidativen Behandlungsmitteln Gesagte.

An das erfindungsgemäße Verfahren zur oxidativen Färbung und/oder Aufhellung keratinischer Fasern, insbesondere von Humanhaar, können sich optional weitere Haarbehandlungsschritte anschließen, beispielsweise die Applikation eines Conditioners, eines Haarverformungsmittels, beispielsweise eines Glättmittels oder eines Wellmittels, eines weiteren Haarfärbemittels, beispielsweise zum Färben oder Blondieren von Strähnchen, Ausspülschritte und Trocknungsschritte, zum Beispiel das Trockenreiben oder Trockenpressen mit dem Handtuch, Fönen oder das Trocknen mit einer Trockenhaube.

### Mischungsverhältnisse von erfindungsgemäßem oxidativen Behandlungsmittel und Zusammensetzung (B)

Es hat sich erfindungsgemäß bewährt, wenn das Gewichtsverhältnis von erfindungsgemäßem oxidativen Behandlungsmittel, das Wasser, Wasserstoffperoxid und die Haar schützende Kombination aus mindestens einer Dicarbonsäure mit 2 bis 10 Kohlenstoffatomen und mindestens einer Aminosäure, bevorzugt mindestens einer Aminosäure der Formel (VI), enthält und einen pH-Wert im Bereich von 2,5 - 6,5, bevorzugt im Bereich von 3 - 5,5, besonders bevorzugt im Bereich von 3,5 bis 5,0, aufweist, jeweils bei 20°C gemessen, zur Zusammensetzung (B), enthaltend mindestens ein Alkalisierungsmittel, ausgewählt aus Ammoniumhydroxid, Monoethanolamin und Natriumsilikaten sowie Mischungen hiervon, ggf. Wasser und und ggf. mindestens ein Oxidationsfarbstoffvorprodukt und/oder mindestens einen direktziehenden Farbstoff, im Bereich von 10:1 bis 1:4, bevorzugt 5:1 bis 1:3, besonders bevorzugt 4:1 bis 1:2, außerordentlich bevorzugt 2:1 bis 1:1 liegt.

Für das erfindungsgemäße Verfahren zur oxidativen Färbung und/oder Aufhellung keratinischer Fasern, insbesondere von Humanhaar, mit dem aus den mindestens zwei vorstehend genannten Zusammensetzungen (A) und (B) und seine bevorzugten Ausführungsformen gilt mutatis mutandis das zu den erfindungsgemäßen und erfindungsgemäß bevorzugten oxidativen Behandlungsmitteln Gesagte.

### Beispiele

Die nachfolgenden Beispiele für erfindungsgemäße oxidative Behandlungsmittel sollen die Erfindung verdeutlichen, ohne sie hierauf zu beschränken.

**Tabelle 1: Oxidative Behandlungsmittel (Developer) für oxidative Haarfarben und Blondierungsmittel (alle Mengenangaben in Gew.-%)**

| Inhaltsstoff | Nr. 1 | Nr. 2 | Nr. 3 | Nr. 4 | Nr. 5 |
|---|---|---|---|---|---|
| Cetearyl Alcohol | 2,8 | 2,2 | 5 | 5 | 5 |
| PEG-40 Castor Oil | 0,6 | 0,4 | 1,5 | 1,5 | 1,5 |
| Sodium Cetearyl Sulfate | 0,4 | 0,3 | 1,0 | 1,0 | 1,0 |
| Paraffinöl | 21,0 | 13,0 | 31,0 | - | - |
| Isopropylmyristat | - | - | - | 15,0 | 31,0 |
| Etidronsäure | 0,15 | 0,15 | 0,15 | 0,15 | 0,15 |
| L-Arginin | 0,4 | 0,4 | - | 0,4 | - |
| L-Lysinhydrochlorid | - | - | 0,7 | - | 0,7 |
| Bernsteinsäure | 1,0 | 1,0 | 1,0 | 1,0 | 1,0 |
| Dinatriumpyrophosphat | 0,1 | 0,1 | 0,1 | 0,1 | 0,1 |
| Dipicolinsäure | 0,1 | 0,1 | 0,1 | 0,1 | 0,1 |
| Natriumbenzoat | 0,04 | 0,04 | 0,04 | 0,04 | 0,04 |
| Kaliumhydroxid | 0,12 | 0,12 | 0,12 | 0,12 | 0,12 |
| Wasserstoffperoxid | 6,0 | 9,0 | 12,0 | 9,0 | 12,0 |
| Wasser (demineralisiert) | ad 100 | ad 100 | ad 100 | ad 100 | ad 100 |

**Tabelle 2: Oxidative Behandlungsmittel (Developer) für oxidative Haarfarben und Blondierungsmittel (alle Mengenangaben in Gew.-%)**

| Inhaltsstoff | Nr. 1 | Nr. 2 | Nr. 3 | Nr. 4 | Nr. 5 |
|---|---|---|---|---|---|
| Cetearyl Alcohol | 2,8 | 2,2 | 5 | 5 | 5 |
| PEG-40 Castor Oil | 0,6 | 0,4 | 1,5 | - | 1,5 |
| Sodium Cetearyl Sulfate | 0,4 | 0,3 | 1,0 | - | - |
| Paraffinöl | 21,0 | 13,0 | 31,0 | - | - |
| Isopropylmyristat | - | - | - | 15,0 | 31,0 |
| Trideceth-2 carboxamide MEA | - | - | - | 2,0 | 2,0 |
| Etidronsäure | 0,15 | 0,15 | 0,15 | 0,15 | 0,15 |
| L-Arginin | 0,4 | 0,4 | - | 0,4 | - |
| L-Lysinhydrochlorid | - | - | 0,7 | - | 0,7 |
| Maleinsäure | 1,0 | 1,0 | - | 1,0 | 1,0 |
| Fumarsäure | - | - | 1,0 | - | - |
| Dinatriumpyrophosphat | 0,1 | 0,1 | 0,1 | 0,1 | 0,1 |
| Dipicolinsäure | 0,1 | 0,1 | 0,1 | 0,1 | 0,1 |
| Natriumbenzoat | 0,04 | 0,04 | 0,04 | 0,04 | 0,04 |
| Kaliumhydroxid | 0,12 | 0,12 | 0,12 | 0,12 | 0,12 |
| Wasserstoffperoxid | 6,0 | 9,0 | 12,0 | 9,0 | 12,0 |
| Wasser (demineralisiert) | ad 100 | ad 100 | ad 100 | ad 100 | ad 100 |

**Tabelle 3: Oxidative Behandlungsmittel (Developer) für oxidative Haarfarben und Blondierungsmittel (alle Mengenangaben in Gew.-%)**

| Inhaltsstoff | Nr. 1 | Nr. 2 | Nr. 3 | Nr. 4 | Nr. 5 |
|---|---|---|---|---|---|
| 1,2-Propylenglycol | 2,0 | 2,0 | 2,0 | 2,0 | 2,0 |
| Cetearyl Alcohol | 1,5 | 1,5 | 3,6 | 1,5 | 3,6 |
| Ceteareth-20 | 0,6 | 0,4 | 1,5 | 1,5 | 1,5 |
| Trideceth-2 carboxamide MEA | - | - | 2,0 | - | 2,0 |
| Paraffinöl | 2,0 | 13,0 | 31,0 | - | - |
| Isopropylmyristat | - | - | - | 2,0 | 31,0 |
| Etidronsäure | 0,15 | 0,15 | 0,15 | 0,15 | 0,15 |
| L-Arginin | 0,4 | 0,4 | - | 0,4 | - |
| L-Lysinhydrochlorid | - | - | 0,7 | - | 0,7 |
| Dinatriumsuccinathexahydrat | 1,5 | 2,0 | 2,0 | 2,0 | 1,5 |
| Steardimonium Hydroxypropyl Hydrolyzed Keratin | 0,1 | 0,1 | 0,1 | 0,1 | 0,1 |
| Dinatriumpyrophosphat | 0,1 | 0,1 | 0,1 | 0,1 | 0,1 |
| Dipicolinsäure | 0,1 | 0,1 | 0,1 | 0,1 | 0,1 |
| Natriumbenzoat | 0,04 | 0,04 | 0,04 | 0,04 | 0,04 |
| Kaliumhydroxid | 0,2 | 0,2 | 0,2 | 0,2 | 0,2 |
| Wasserstoffperoxid | 6,0 | 9,0 | 12,0 | 6,0 | 9,0 |
| Wasser (demineralisiert) | ad 100 | ad 100 | ad 100 | ad 100 | ad 100 |

**Tabelle 4: Färbecremes (alle Mengenangaben in Gew.-%)**

| Formulierungsbestandteile (Farbcreme) | 1 | 2 |
|---|---|---|
| Cetylalkohol | 3,6 | 4,6 |
| Behenylalcohol | 2,4 | 3,0 |
| Paraffinum Liquidum | 2,1 | 2,1 |
| Eumulgin B 3 (INCI: Ceteareth-30) | 1,2 | 1,8 |
| Brij S 100 PA (Stearyl alcohol ethoxylated (100 EO)) | 0,6 | 1,0 |
| Cutina GMS (INCI: Glyceryl Stearate) | 0,6 | 0,6 |
| Propylenglycol | 6,0 | 6,0 |
| p-Toluylendiamin Sulfat | 1,50 | 1,50 |
| Resorcin | 0,58 | 0,58 |
| m-Aminophenol | 0,16 | 0,16 |
| 3-Amino-2-methylamino-6-methoxypyridin | 0,05 | 0,05 |
| Kaliumhydroxid (50 %ig) | 0,7 | 0,7 |
| Ethylendiamintetraessigsäure, Tetranatriumsalz | 0,20 | 0,20 |
| Natriumsulfit (wasserfrei) | 0,30 | 0,30 |
| Vitamin C | 0,05 | 0,05 |
| Produkt W 37194 (N,N,N-Trimethyl-3-[(1-oxo-2-propenyl)amino]-1-propanaminiumchloride, polymer with sodium 2-propenoate) (INCI: Acrylamidopropyltrimonium chloride /Acrylate Copolymer), 20 Gew.-%ige wässrige Lösung | 3,75 | 3,0 |
| Ammoniak (25 Gew.-%ige wässrige Lösung) | 5,80 | 5,80 |
| Parfüm | 0,40 | 0,40 |
| Wasser | ad 100 | ad 100 |

Die Farbcremes 1 und 2 gemäß Tabelle 4 entsprechen den in der Beschreibung dargestellten Zusammensetzungen (B). Sie wurden jeweils im Gewichtsverhältnis 1:1 mit einem der oxidativen Behandlungsmittel aus den Tabellen 1, 2 oder 3 vermischt, direkt anschließend auf Humanhaarsträhnen aufgetragen und nach einer Einwirkzeit von 45 Minuten mit Wasser und einem Shampoo ausgespült. Anschließend wurde das Haar zuerst mit einem Handtuch und dann mit einem Fön getrocknet.

**Tabelle 5: Blondierpulver (pulverförmige Zusammensetzung (B))**

| Formulierungsbestandteile | (Gew.-%) |
|---|---|
| Natriumsilikat (SiO₂:Na₂O = 2:1) | 36,00 |
| Magnesiumcarbonat | 12,85 |
| Natriumhexametaphosphat | 0,20 |
| Rohagit S hv (Methylmethacrylat, Methacrylsäure Copolymer) | 1,00 |
| EDTA Dinatriumsalz (Ethylenediaminetetraessigsäure, Dinatriumsalz) | 0,60 |
| Polyquaternium-4 | 0,30 |
| Kieselsäure, hydrophil | 0,40 |
| Glycin | 0,60 |
| Kaliumpersulfat | 32,00 |
| Ammoniumpersulfat | 10,00 |
| CI 77007 (ULTRAMARINES) | 0,15 |
| Dimethicone (85 Gew.-%), Dimethiconol (15 Gew.-%) | 1,50 |
| Paraffinum Liquidum | 3,80 |

Zur Herstellung des anwendungsbereiten Blondierungsmittels wurden jeweils 2 Gewichtsteile eines oxidativen Behandlungsmittels aus den Tabellen 1, 2 oder 3 mit einem Gewichtsteil des Blondierpulvers gemäß Tabelle 5 vermischt, direkt anschließend auf Humanhaarsträhnen aufgetragen und nach einer Einwirkzeit von 50 Minuten mit Wasser und einem Shampoo ausgespült. Anschließend wurde das Haar zuerst mit einem Handtuch und dann mit einem Fön getrocknet.

## Patentansprüche

1. Oxidatives Behandlungsmittel für keratinische Fasern, insbesondere für Humanhaar, enthaltend
a) mindestens eine Dicarbonsäure mit 2 bis 10 Kohlenstoffatomen und/oder mindestens ein Salz dieser Säure(n),
b) mindestens eine Aminosäure,
c) Wasserstoffperoxid, bevorzugt in einer Menge von 0,5 bis 13 Gew.-%, weiter bevorzugt 1 bis 7 Gew.-%, besonders bevorzugt 2 bis 6 Gew.-% und ganz besonders bevorzugt 3 bis 4,5 Gew.-% (berechnet als 100 %-iges H₂O₂), jeweils bezogen auf das Gesamtgewicht des erfindungsgemäßen oxidativen Behandlungsmittels, und
d) Wasser, bevorzugt in einer Menge von 20 bis 95 Gew.-%, bevorzugt 30 bis 90 Gew.-%, besonders bevorzugt 40 bis 85 Gew.-%, außerordentlich bevorzugt 50 bis 80 Gew.-%, jeweils bezogen auf das Gesamtgewicht des erfindungsgemäßen oxidativen Behandlungsmittels,
wobei das oxidative Behandlungsmittel einen pH-Wert im Bereich von 2,5 - 6,5, bevorzugt im Bereich von 3 - 5,5, besonders bevorzugt im Bereich von 3,5 bis 5,0, aufweist, jeweils bei 20°C gemessen.

2. Behandlungsmittel gemäß Anspruch 1, **dadurch gekennzeichnet, dass** die mindestens eine Aminosäure ausgewählt ist aus Aminosäuren der Formel (VI) 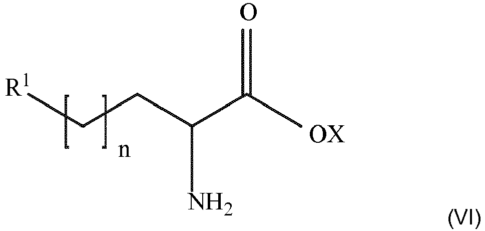 worin
X für ein Wasserstoffatom oder ein ein- oder zweiwertiges Kation steht;
n für null, 1, 2 oder 3 steht;
R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens ein Salz dieser Aminosäure.

3. Behandlungsmittel gemäß Anspruch 1 oder 2, **dadurch gekennzeichnet, dass** die mindestens eine Dicarbonsäure mit 2 bis 10 Kohlenstoffatomen ausgewählt ist aus Maleinsäure, Fumarsäure, Bernsteinsäure, Äpfelsäure, Oxalsäure, Malonsäure, Adipinsäure, Pimelinsäure, Korksäure, Azelainsäure, Sebacinsäure, D-Weinsäure, L-Weinsäure, meso-Weinsäure, Traubensäure, alpha-Ketoglutarsäure, beta-Ketoglutarsäure sowie Mischungen dieser Säuren, bevorzugt ausgewählt aus Bernsteinsäure, Äpfelsäure, Maleinsäure und Fumarsäure, besonders bevorzugt ausgewählt aus Bernsteinsäure.

4. Behandlungsmittel gemäß einem der Ansprüche 1 bis 3, **dadurch gekennzeichnet, dass** die mindestens eine Dicarbonsäure mit 2 bis 10 Kohlenstoffatomen in einer Gesamtmenge von 0,1 bis 5 Gew.-%, bevorzugt 0,2 bis 4 Gew.-%, besonders bevorzugt 0,5 bis 2 Gew.-%, enthalten ist, jeweils umgerechnet auf die undissoziierte Säure und bezogen auf das Gewicht des Behandlungsmittels.

5. Behandlungsmittel gemäß einem der Ansprüche 2 bis 4, **dadurch gekennzeichnet, dass** die mindestens eine Aminosäure der Formel (VI) ausgewählt ist aus Arginin, Lysin, Histidin, Asparagin, Glutamin, Cystein, Methionin, Tryptophan sowie Mischungen hiervon, besonders bevorzugt Mischungen aus Arginin und Lysin.

6. Behandlungsmittel gemäß einem der Ansprüche 1 bis 5, **dadurch gekennzeichnet, dass** die mindestens eine Aminosäure in einer Gesamtmenge von 0,05 bis 3 Gew.-%, bevorzugt 0,1 bis 2 Gew.-%, besonders bevorzugt 0,2 bis 1,2 Gew.-%, enthalten ist, jeweils umgerechnet auf die undissoziierte Säure und bezogen auf das Gewicht des Behandlungsmittels.

7. Behandlungsmittel gemäß einem der Ansprüche 1 - 6, **dadurch gekennzeichnet, dass** mindestens ein Chelatbildner enthalten ist, bevorzugt in einer Gesamtmenge von 0,01 - 2,5 Gew.-%, besonders bevorzugt in einer Gesamtmenge von 0,1 - 1,5 Gew.-%, außerordentlich bevorzugt in einer Gesamtmenge von 0,2 - 0,6 Gew.-%, jeweils bezogen auf das Gewicht des oxidativen Behandlungsmittels.

8. Behandlungsmittel gemäß einem der Ansprüche 1 - 7, **dadurch gekennzeichnet, dass** mindestens eine Verbindung der allgemeinen Formel (III) enthalten ist, 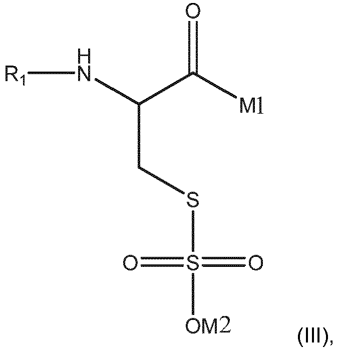 wobei
R1 für ein Wasserstoffatom oder für ein Strukturelement der Formel (IV) steht 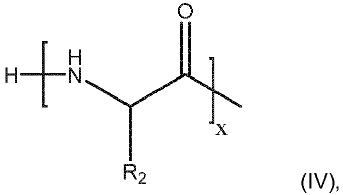 wobei
x für eine ganze Zahl von 1 bis 100 steht,
der Rest R2 in jedem der Strukturelemente der Formel (IV) jeweils unabhängig vom vorangegangenen Strukturelement der Formel (IV) gewählt werden kann,
R2 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (V) 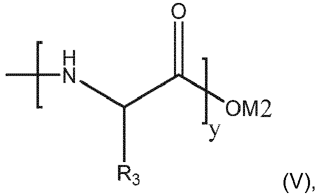 wobei
y für eine ganze Zahl von 1 bis 100 steht,
der Rest R3 in jedem der Strukturelemente der Formel (V) jeweils unabhängig vom vorangegangenen Strukturelement der Formel (V) gewählt werden kann,
R3 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M2 steht für ein Wasserstoffatom, ein Äquivalent eines ein oder mehrwertigen Kations oder ein Ammoniumion (NH₄)⁺,
wobei bevorzugt eine oder mehrere Verbindungen der vorstehend aufgeführten Formel (III) in einer Gesamtmenge von 0,001 bis 2,5 Gew.-% weiter bevorzugt von 0,01 bis 1,0 Gew.-% und besonders bevorzugt von 0,02 bis 0,1 Gew.-% enthalten sind, jeweils bezogen auf das Gewicht des Behandlungsmittels.

9. Behandlungsmittel gemäß einem der Ansprüche 1 - 8, **dadurch gekennzeichnet, dass** mindestens ein Fettsäureamid mit der Formel (AMID-I) enthalten ist, bevorzugt in einer Gesamtmenge von 0,1 bis 10 Gew.-%, besonders bevorzugt 0,2 bis 5 Gew.-%, außerordentlich bevorzugt 0,4 - 2 Gew.-%, jeweils bezogen auf das Gewicht des Behandlungsmittels, 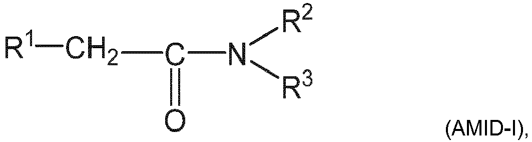 worin
- R¹ ausgewählt ist aus linearen und verzweigten C4-C22-Alkylgruppen, die gesättigt oder ungesättigt sind, wobei die Alkyl- und Alkenylgruppen unsubstituiert sind oder mit Sauerstoffatomen, Stickstoffatomen, Hydroxylgruppen, Ethergruppen, Oxyalkylengruppen, Polyoxyalkylengruppen, Carbonsäuregruppen, Amingruppen, Amidgruppen, Halogen-enthaltenden Gruppen, Estergruppen, Siloxangruppen oder Polysiloxangruppen substituiert sein können,
und alkoxylierten Alkylgruppen der Formeln: 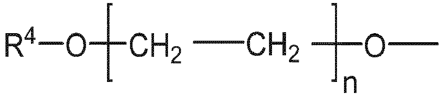 und 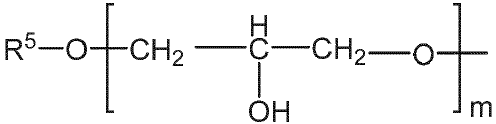 worin:
- R⁴ und R⁵, die gleich oder verschieden sein können, ausgewählt sind aus linearen und verzweigten C4-C22-Alkylgruppen, die gesättigt oder ungesättigt sind,
- n eine ganze Zahl im Bereich von 1 bis 10, bevorzugt im Bereich von 1 bis 4 ist und
- m eine ganze Zahl im Bereich von 1 bis 6 liegt und
- R² und R³, die gleich oder verschieden sein können, jeweils unabhängig voneinander ausgewählt sind aus H; linearen und verzweigten C1-C22-Alkylgruppen, die gesättigt oder ungesättigt sind, wobei die Alkyl- und Alkenylgruppen unsubstituiert sind oder mit Sauerstoffatomen, Stickstoffatomen, Hydroxylgruppen, Ethergruppen, Oxyalkylengruppen, insbesondere Oxyethylengruppen, Polyoxyalkylengruppen, insbesondere Polyoxyethylengruppen, Carbonsäuregruppen, Amingruppen, Alkylamingruppen, insbesondere C1-C3-Alkylamingruppen, Dialkylamingruppen, insbesondere Di-(C1-C3)-alkylamingruppen, substituierten und nicht substituierten 4,5-Dihydroimidazoliumgrupppen, Amidgruppen, Halogen enthaltenden Gruppen, Estergruppen, Siloxangruppen oder Polysiloxangruppen substituiert sind.

10. Verfahren zur oxidativen Färbung und/oder Aufhellung keratinischer Fasern, insbesondere von Humanhaar, bei dem ein Behandlungsmittel gemäß einem der Ansprüche 1 - 9 auf die keratinischen Fasern, insbesondere auf das Humanhaar, aufgetragen und ggf. nach einer Einwirkzeit von 0,1 bis 60 Minuten, bevorzugt 1 bis 50 Minuten, besonders bevorzugt 10 bis 45 Minuten, außerordentlich bevorzugt 30 bis 45 Minuten, wieder ausgespült wird.

11. Verfahren zur oxidativen Färbung und/oder Aufhellung keratinischer Fasern, insbesondere von Humanhaar, das folgende Verfahrensschritte umfasst:
I. Bereitstellen eines oxidativen Behandlungsmittels (A), enthaltend
a) mindestens eine Dicarbonsäure mit 2 bis 10 Kohlenstoffatomen und/oder mindestens ein Salz dieser Säure(n), die bevorzugt ausgewählt ist aus Maleinsäure, Fumarsäure, Bernsteinsäure, Äpfelsäure, Oxalsäure, Malonsäure, Adipinsäure, Pimelinsäure, Korksäure, Azelainsäure, Sebacinsäure, D-Weinsäure, L-Weinsäure, meso-Weinsäure, Traubensäure, alpha-Ketoglutarsäure, beta-Ketoglutarsäure sowie Mischungen dieser Säuren, besonders bevorzugt ausgewählt aus Bernsteinsäure, Maleinsäure und Fumarsäure, außerordentlich bevorzugt ausgewählt aus Bernsteinsäure, weiterhin
b) mindestens eine Aminosäure, die bevorzugt ausgewählt ist aus Aminosäuren der Formel (VI) 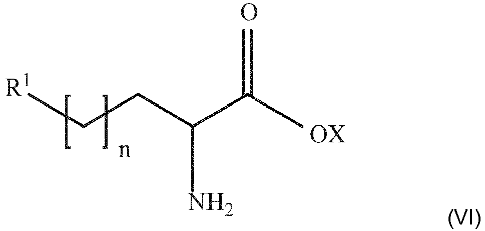 worin
X für ein Wasserstoffatom oder ein ein- oder zweiwertiges Kation steht;
n für null, 1, 2 oder 3 steht;
R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe - SCH₃, oder mindestens ein Salz dieser Aminosäure,
c) Wasserstoffperoxid,
d) Wasser,
e) wobei das oxidative Behandlungsmittel einen pH-Wert im Bereich von 2,5 - 6,5, bevorzugt im Bereich von 3 - 5,5, besonders bevorzugt im Bereich von 3,5 bis 5,0, aufweist, jeweils bei 20°C gemessen,
f) optional weiterhin mindestens eine Substanz, die ausgewählt ist aus
- Verbindungen der allgemeinen Formel (III), 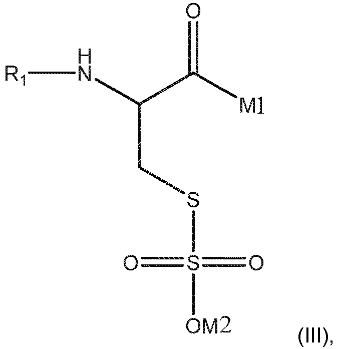 wobei
R1 für ein Wasserstoffatom oder für ein Strukturelement der Formel (IV) steht 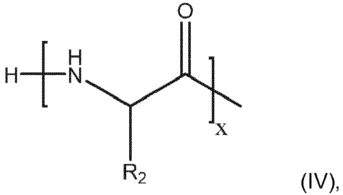 wobei
x für eine ganze Zahl von 1 bis 100 steht,
der Rest R2 in jedem der Strukturelemente der Formel (IV) jeweils unabhängig vom vorangegangenen Strukturelement der Formel (IV) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)-methyl-Gruppe,
M1 steht für die Gruppierung -OM2 oder für ein Strukturelement der Formel (V) 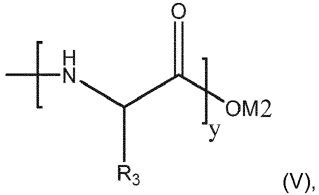
wobei
y für eine ganze Zahl von 1 bis 100 steht,
der Rest R3 in jedem der Strukturelemente der Formel (V) jeweils unabhängig vom vorangegangenen Strukturelement der Formel (V) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)-methyl-Gruppe,
M2 steht für ein Wasserstoffatom, ein Äquivalent eines ein oder mehrwertigen Kations oder ein Ammoniumion (NH₄)⁺,
- mindestens einem Chelatbildner, bevorzugt in einer Gesamtmenge von 0,01 - 2,5 Gew.-%, besonders bevorzugt in einer Gesamtmenge von 0,1 - 1,5 Gew.-%, außerordentlich bevorzugt in einer Gesamtmenge von 0,2 - 0,6 Gew.-%, jeweils bezogen auf das Gewicht des oxidativen Behandlungsmittels (A), und
- mindestens einem Fettsäureamid mit der Formel (AMID-I), bevorzugt in einer Gesamtmenge von 0,1 bis 10 Gew.-%, besonders bevorzugt 0,2 bis 5 Gew.-%, außerordentlich bevorzugt 0,4 - 2 Gew.-%, jeweils bezogen auf das Gewicht des oxidativen Behandlungsmittels (A), 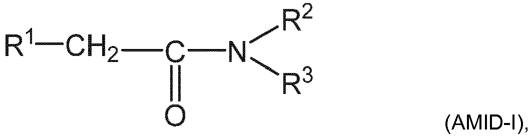 worin
R¹ ausgewählt ist aus linearen und verzweigten C4-C22-Alkylgruppen, die gesättigt oder ungesättigt sind, wobei die Alkyl- und Alkenylgruppen unsubstituiert sind oder mit Sauerstoffatomen, Stickstoffatomen, Hydroxylgruppen, Ethergruppen, Oxyalkylengruppen, Polyoxyalkylengruppen, Carbonsäuregruppen, Amingruppen, Amidgruppen, Halogen-enthaltenden Gruppen, Estergruppen, Siloxangruppen oder Polysiloxangruppen substituiert sein können,
und alkoxylierten Alkylgruppen der Formeln: 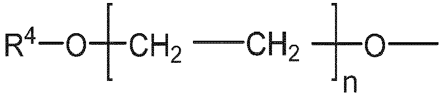 und 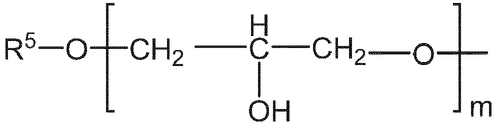 worin:
R⁴ und R⁵, die gleich oder verschieden sein können, ausgewählt sind aus linearen und verzweigten C4-C22-Alkylgruppen, die gesättigt oder ungesättigt sind,
n eine ganze Zahl im Bereich von 1 bis 10, bevorzugt im Bereich von 1 bis 4 ist und
m eine ganze Zahl im Bereich von 1 bis 6 liegt und
R² und R³, die gleich oder verschieden sein können, jeweils unabhängig voneinander ausgewählt sind aus H; linearen und verzweigten C1-C22-Alkylgruppen, die gesättigt oder ungesättigt sind, wobei die Alkyl- und Alkenylgruppen unsubstituiert sind oder mit Sauerstoffatomen, Stickstoffatomen, Hydroxylgruppen, Ethergruppen, Oxyalkylengruppen, insbesondere Oxyethylengruppen, Polyoxyalkylengruppen, insbesondere Polyoxyethylengruppen, Carbonsäuregruppen, Amingruppen, Alkylamingruppen, insbesondere C1-C3-Alkylamingruppen, Dialkylamingruppen, insbesondere Di-(C1-C3)-alkylamingruppen, substituierten und nicht substituierten 4,5-Dihydroimidazoliumgrupppen, Amidgruppen, Halogen enthaltenden Gruppen, Estergruppen, Siloxangruppen oder Polysiloxangruppen substituiert sind,
II. Bereitstellen einer Zusammensetzung (B), enthaltend
g) mindestens ein Alkalisierungsmittel, ausgewählt aus Ammoniumhydroxid, Monoethanolamin und Natriumsilikaten sowie Mischungen hiervon,
h) ggf. Wasser und
i) ggf. mindestens ein Oxidationsfarbstoffvorprodukt und/oder mindestens einen direktziehenden Farbstoff,
wobei die Zusammensetzung (B) bevorzugt einen pH-Wert im Bereich von 6,5 bis 12,0, bevorzugt 8 - 11,5, besonders bevorzugt 8,5 bis 11,0, aufweist, jeweils gemessen bei 20°C,
III. Vermischen der Zusammensetzungen (A) und (B) miteinander, wobei das Gewichtsverhältnis von (A) zu (B) bevorzugt im Bereich von 10:1 bis 1:4, bevorzugt im Bereich von 5:1 bis 1:3, besonders bevorzugt im Bereich von 4:1 bis 1:2, außerordentlich bevorzugt im Bereich von 2:1 bis 1:1 liegt, direkt anschließend
IV. Auftragen der Mischung aus (A) und (B) auf die keratinischen Fasern, insbesondere auf das Humanhaar, und
V. Ausspülen nach einer Einwirkzeit von 0,1 bis 60 Minuten, bevorzugt 1 bis 50 Minuten, besonders bevorzugt 10 bis 45 Minuten, außerordentlich bevorzugt 30 bis 45 Minuten,
VI. ggf. weitere Haarbehandlungen, wie Verformen, Konditionieren und/oder Trocknen.

12. Verfahren gemäß Anspruch 10 oder 11, **dadurch gekennzeichnet, dass** das oxidative Behandlungsmittel (A) die mindestens eine Dicarbonsäure mit 2 bis 10 Kohlenstoffatomen in einer Gesamtmenge von 0,1 bis 5 Gew.-%, bevorzugt 0,2 bis 4 Gew.-%, besonders bevorzugt 0,5 bis 2 Gew.-%, enthält, jeweils umgerechnet auf die undissoziierte Säure und bezogen auf das Gewicht des Behandlungsmittels (A).

13. Verfahren gemäß einem der Ansprüche 10 bis 12, **dadurch gekennzeichnet, dass** das oxidative Behandlungsmittel (A) die mindestens eine Aminosäure in einer Gesamtmenge von 0,05 bis 3 Gew.-%, bevorzugt 0,1 bis 2 Gew.-%, besonders bevorzugt 0,2 bis 1,2 Gew.-%, enthält, jeweils umgerechnet auf die undissoziierte Säure und bezogen auf das Gewicht des oxidativen Behandlungsmittels (A).

14. Verfahren gemäß einem der Ansprüche 11 bis 13, **dadurch gekennzeichnet, dass** die Zusammensetzung (B) pulverförmig ist und mindestens ein Persulfatsalz und/oder mindestens ein Percarbonatsalz enthält.

## Claims

1. An oxidative treatment agent for keratin fibers, in particular for human hair, comprising:
a) at least one dicarboxylic acid having 2 to 10 carbon atoms and/or at least one salt of this acid/these acids,
b) at least one amino acid,
c) hydrogen peroxide, preferably in an amount of 0.5 to 13 wt.%, more preferably 1 to 7 wt.%, particularly preferably 2 to 6 wt.%, and especially particularly preferably 3 to 4.5 wt.% (calculated as 100% H₂O₂), in each case based on the total weight of the oxidative treatment agent according to the invention, and
d) water, preferably in an amount of 20 to 95 wt.%, preferably 30 to 90 wt.%, particularly preferably 40 to 85 wt.%, and exceptionally preferably 50 to 80 wt.%, in each case based on the total weight of the oxidative treatment agent according to the invention,
wherein the oxidative treatment agent has a pH value in the range of 2.5 to 6.5, preferably in the range of 3 to 5.5, and particularly preferably in the range of 3.5 to 5.0, each measured at 20°C.

2. The treatment agent according to claim 1, **characterized in that** the at least one amino acid is selected from amino acids of formula (VI) 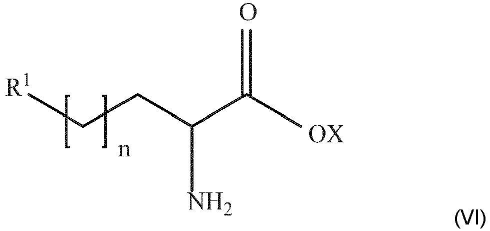 where
X denotes a hydrogen atom or a monovalent or divalent cation;
n denotes zero, 1, 2 or 3;
R¹ denotes a group selected from an amino group, a guanidine group, a (1*H*-imidazole-4-yl) group, a carboxylic acid amid group -CONH₂, a 1*H*-indole-3-yl group, a thiol group -SH, and a methylsulfanyl group -SCH₃, or at least one salt of these amino acids.

3. The treatment agent according to claim 1 or 2, **characterized in that** the at least one dicarboxylic acid having 2 to 10 carbon atoms is selected from maleic acid, fumaric acid, succinic acid, oxalic acid, malonic acid, adipic acid, pimelic acid, suberic acid, azelaic acid, sebacic acid, and mixtures of these acids, preferably selected from succinic acid, maleic acid and fumaric acid, and particularly preferably selected from succinic acid.

4. The treatment agent according to any one of claims 1 to 3, **characterized in that** the at least one dicarboxylic acid having 2 to 10 carbon atoms is present in a total amount of 0.1 to 5 wt.%, preferably 0.2 to 4 wt.%, and particularly preferably 0.5 to 2 wt.%, each converted to the undissociated acid and based on the weight of the treatment agent.

5. The treatment agent according to any one of claims 2 to 4, **characterized in that** the at least one amino acid of formula (VI) is selected from arginine, lysine, histidine, asparagine, glutamine, cysteine, methionine, tryptophan, and mixtures thereof, and particularly preferably mixtures of arginine and lysine.

6. The treatment agent according to any one of claims 1 to 5, **characterized in that** the at least one amino acid is present in a total amount of 0.05 to 3 wt.%, preferably 0.1 to 2 wt.%, and particularly preferably 0.2 to 1.2 wt.%, each converted to the undissociated acid and based on the weight of the treatment agent.

7. The treatment agent according to any one of claims 1 to 6, **characterized in that** at least one chelating agent is present, preferably in a total amount of 0.01 to 2.5 wt.%, particularly preferably in a total amount of 0.1 to 1.5 wt.%, and exceptionally preferably in a total amount of 0.2 to 0.6 wt.%, in each case based on the weight of the oxidative treatment agent.

8. The treatment agent according to one of claims 1 to 7, **characterized in that** at least one compound of the general formula (III) is present, 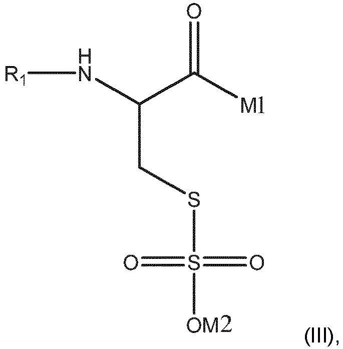 wherein
R1 denotes a hydrogen atom or a structural element of formula (IV) 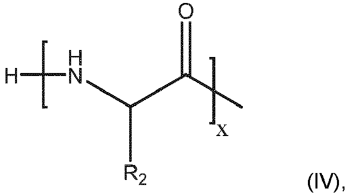 wherein
a denotes an integer from 1 to 100,
the group R2 in each of the structural elements of formula (IV) can each be selected independently of the preceding structural element of formula (IV),
R2 denotes a hydrogen atom, a methyl group, an isopropyl group, a 2-methylpropyl group, a 1-methylpropyl group, a benzyl group, a 4-hydroxybenzyl group, a hydroxymethyl group, a 1-hydroxyethyl group, a 4-aminobutyl group, a 3-carbamimidamidopropyl group, a 2-carboxyethyl group, a carboxymethyl group, a 2-carbamoylethyl group, a carbamoylmethyl group, a sulfanylmethyl group, a 2-(methylsulfanyl)ethyl group, a 1H-imidazole-4-ylmethyl group, a 1H-indole-3-ylmethyl group or a (sulfosulfanyl)methyl group,
M1 denotes the group -OM2 or a structural element of formula (V), 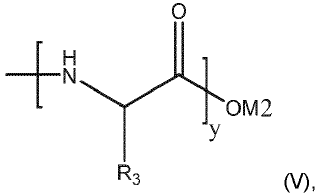 wherein
y denotes an integer from 1 to 100,
the group R3 in each of the structural elements of formula (V) can each be selected independently of the preceding structural element of formula (V),
R3 denotes a hydrogen atom, a methyl group, an isopropyl group, a 2-methylpropyl group, a 1-methylpropyl group, a benzyl group, a 4-hydroxybenzyl group, a hydroxymethyl group, a 1-hydroxyethyl group, a 4-aminobutyl group, a 3-carbamimidamidopropyl group, a 2-carboxyethyl group, a carboxymethyl group, a 2-carbamoylethyl group, a carbamoylmethyl group, a sulfanylmethyl group, a 2-(methylsulfanyl)ethyl group, a 1H-imidazole-4-ylmethyl group, a 1H-indole-3-ylmethyl group or a (sulfosulfanyl)methyl group,
M2 denotes a hydrogen atom, an equivalent of a monovalent or polyvalent cation, or an ammonium ion (NH₄)⁺,
wherein preferably one or more compounds of the above-described formula (III) is present in a total amount of 0.001 to 2.5 wt.%, more preferably of 0.01 to 1.0 wt.%, and particularly preferably of 0.02 to 0.1 wt.%, in each case based on the weight of the treatment agent.

9. The treatment agent according to any one of claims 1 to 8, **characterized in that** at least one fatty acid amide of formula (AMID-I) is present, preferably in a total amount of 0.1 to 10 wt.%, particularly preferably 0.2 to 5 wt.%, and exceptionally preferably 0.4 to 2 wt.%, in each case based on the weight of the treatment agent, 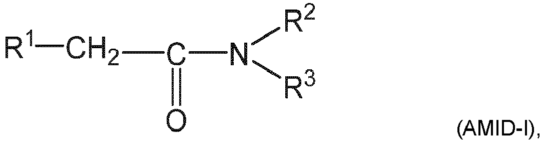 where
- R¹ is selected from linear or branched C4 to C22 alkyl groups, which are saturated or unsaturated, wherein the alkyl and alkenyl groups are unsubstituted or may be substituted with oxygen atoms, nitrogen atoms, hydroxyl groups, ether groups, oxyalkylene groups, polyoxyalkylene groups, carboxylic acid groups, amine groups, amide groups, halogen-containing groups, ester groups, siloxane groups, or polysiloxane groups,
and alkoxylated alkyl groups of formulas: 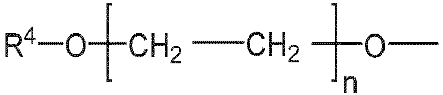 and 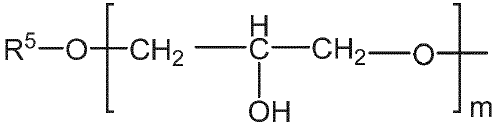 where:
- R⁴ and R⁵, which can be the same or different, are selected from linear and branched C4 to C22 alkyl groups, which are saturated or unsaturated,
- n is an integer in the range from 1 to 10, and preferably in the range from 1 to 4; and
- m is an integer in the range from 1 to 6; and
- R² and R³, which may be the same or different, are each independently of one another selected from H; linear or branched C1 to C22 alkyl groups, which are saturated or unsaturated, wherein the alkyl and alkenyl groups are unsubstituted or substituted with oxygen atoms, nitrogen atoms, hydroxyl groups, ether groups, oxyalkylene groups, and in particular oxyethylene groups, polyoxyalkylene groups, and in particular polyoxyethylene groups, carboxylic acid groups, amine groups, alkylamine groups, and in particular C1 to C3 alkylamine groups, dialkylamine groups, and in particular di-(C1-C3)-alkylamine groups, substituted and non-substituted 4,5-dihydroimidazole groups, amide groups, halogen-containing groups, ester groups, siloxane groups, or polysiloxane groups.

10. A method for oxidatively coloring and/or lightening keratin fibers, and in particular human hair, wherein a treatment agent according to any one of claims 1 to 9 is applied to the keratin fibers, and in particular to the human hair, and optionally is rinsed off again after an exposure time of 0.1 to 60 minutes, preferably 1 to 50 minutes, particularly preferably 10 to 45 minutes, and exceptionally preferably 30 to 45 minutes.

11. A method for oxidatively coloring and/or lightening keratin fibers, and in particular human hair, comprising the following method steps:
I. providing an oxidative treatment agent (A), comprising
a) at least one dicarboxylic acid having 2 to 10 carbon atoms and/or at least one salt of this acid/these acids, which is preferably selected from maleic acid, fumaric acid, succinic acid, oxalic acid, malonic acid, adipic acid, pimelic acid, suberic acid, azelaic acid, sebacic acid, and mixtures of these acids, particularly preferably selected from succinic acid, maleic acid and fumaric acid, and exceptionally preferably selected from succinic acid, furthermore
b) at least one amino acid, which is preferably selected from amino acids of formula (VI) 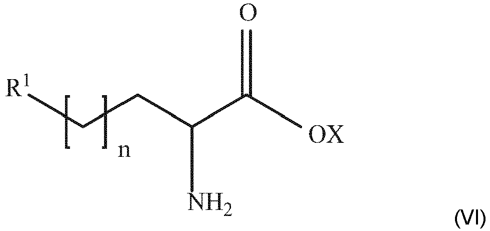 where
X denotes a hydrogen atom or a monovalent or divalent cation;
n denotes zero, 1, 2 or 3;
R¹ denotes a group selected from an amino group, a guanidine group, a (1*H*-imidazole-4-yl) group, a carboxylic acid amid group -CONH₂, a 1*H*-indole-3-yl group, a thiol group -SH, and a methylsulfanyl group -SCH₃, or at least one salt of these amino acids,
c) hydrogen peroxide,
d) water,
e) wherein the oxidative treatment agent has a pH value in the range of 2.5 to 6.5, preferably in the range of 3 to 5.5, and particularly preferably in the range of 3.5 to 5.0, each measured at 20°C,
f) optionally furthermore at least one substance selected from
- compounds of the general formula (III), 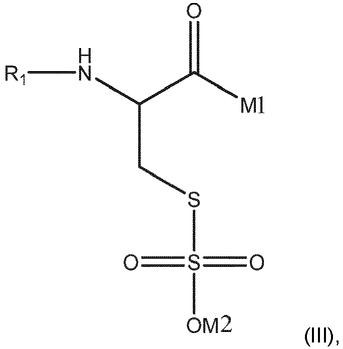 wherein
R1 denotes a hydrogen atom or a structural element of formula (IV) 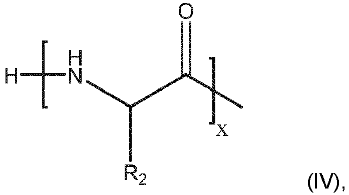 wherein
a denotes an integer from 1 to 100,
the group R2 in each of the structural elements of formula (IV) can each be selected independently of the preceding structural element of formula (IV),
R2 denotes a hydrogen atom, a methyl group, an isopropyl group, a 2-methylpropyl group, a 1-methylpropyl group, a benzyl group, a 4-hydroxybenzyl group, a hydroxymethyl group, a 1-hydroxyethyl group, a 4-aminobutyl group, a 3-carbamimidamidopropyl group, a 2-carboxyethyl group, a carboxymethyl group, a 2-carbamoylethyl group, a carbamoylmethyl group, a sulfanylmethyl group, a 2-(methylsulfanyl)ethyl group, a 1H-imidazole-4-ylmethyl group, a 1H-indole-3-ylmethyl group or a (sulfosulfanyl)methyl group,
M1 denotes the group -OM2 or a structural element of formula (V), 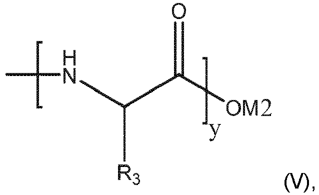 wherein
y denotes an integer from 1 to 100,
the group R3 in each of the structural elements of formula (V) can each be selected independently of the preceding structural element of formula (V),
R3 denotes a hydrogen atom, a methyl group, an isopropyl group, a 2-methylpropyl group, a 1-methylpropyl group, a benzyl group, a 4-hydroxybenzyl group, a hydroxymethyl group, a 1-hydroxyethyl group, a 4-aminobutyl group, a 3-carbamimidamidopropyl group, a 2-carboxyethyl group, a carboxymethyl group, a 2-carbamoylethyl group, a carbamoylmethyl group, a sulfanylmethyl group, a 2-(methylsulfanyl)ethyl group, a 1H-imidazole-4-ylmethyl group, a 1H-indole-3-ylmethyl group or a (sulfosulfanyl)methyl group,
M2 denotes a hydrogen atom, an equivalent of a monovalent or polyvalent cation, or an ammonium ion (NH₄)⁺,
- at least one chelating agent, preferably in a total amount of 0.01 to 2.5 wt.%, particularly preferably in a total amount of 0.1 to 1.5 wt.%, and exceptionally preferably in a total amount of 0.2 to 0.6 wt.%, in each case based on the weight of the oxidative treatment agent (A), and
- at least one fatty acid amide of formula (AMID-I), preferably in a total amount of 0.1 to 10 wt.%, particularly preferably 0.2 to 5 wt.%, and exceptionally preferably 0.4 to 2 wt.%, in each case based on the weight of the oxidative treatment agent (A), 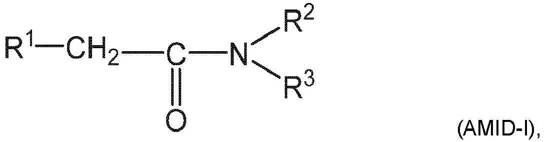 where
R¹ is selected from linear and branched C4 to C22 alkyl groups, which are saturated or unsaturated, wherein the alkyl and alkenyl groups are unsubstituted or may be substituted with oxygen atoms, nitrogen atoms, hydroxyl groups, ether groups, oxyalkylene groups, polyoxyalkylene groups, carboxylic acid groups, amine groups, amide groups, halogen-containing groups, ester groups, siloxane groups, or polysiloxane groups,
and alkoxylated alkyl groups of formulas: 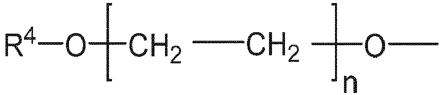 and 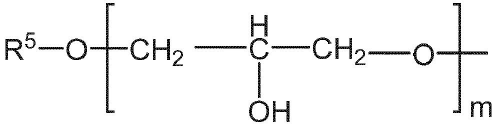 where:
R⁴ and R⁵, which can be the same or different, are selected from linear or branched C4 to C22 alkyl groups, which are saturated or unsaturated,
n is an integer in the range from 1 to 10, and preferably in the range from 1 to 4; and
m is an integer in the range from 1 to 6; and
R² and R³, which may be the same or different, are each independently of one another selected from H; linear or branched C1 to C22 alkyl groups, which are saturated or unsaturated, wherein the alkyl and alkenyl groups are unsubstituted or substituted with oxygen atoms, nitrogen atoms, hydroxyl groups, ether groups, oxyalkylene groups, and in particular oxyethylene groups, polyoxyalkylene groups, and in particular polyoxyethylene groups, carboxylic acid groups, amine groups, alkylamine groups, and in particular C1 to C3 alkylamine groups, dialkylamine groups, and in particular di-(C1-C3)-alkylamine groups, substituted or non-substituted 4,5-dihydroimidazole groups, amide groups, halogen-containing groups, ester groups, siloxane groups, or polysiloxane groups,
II. providing a composition (B), comprising
g) at least one alkalizing agent, selected from ammonium hydroxide, monoethanolamine and sodium silicates, and mixtures thereof,
h) optionally water, and
i) optionally at least one oxidation dye precursor and/or at least one direct dye,
wherein composition (B) preferably has a pH value in the range of 6.5 to 12.0, preferably 8 to 11.5, and particularly preferably 8.5 to 11.0, each measured at 20°C,
III. mixing compositions (A) and (B) with each other, wherein the weight ratio of (A) to (B) is preferably in the range of 10:1 to 1:4, preferably in the range of 5:1 to 1:3, particularly preferably in the range of 4:1 to 1:2, and exceptionally preferably in the range of 2:1 to 1:1, directly thereafter
IV. applying the mixture of (A) and (B) to the keratin fibers, and in particular to the human hair, and
V. rinsing after an exposure time of 0.1 to 60 minutes, preferably 1 to 50 minutes, particularly preferably 10 to 45 minutes, and exceptionally preferably 30 to 45 minutes, and
VI. optionally further heat treatments, such as styling, conditioning and/or drying.

12. The method according to claim 10 or 11, **characterized in that** the oxidative treatment agent (A) comprises the at least one dicarboxylic acid having 2 to 10 carbon atoms in a total amount of 0.1 to 5 wt.%, preferably 0.2 to 4 wt.%, and particularly preferably 0.5 to 2 wt.%, each converted to the undissociated acid and based on the weight of the treatment agent (A).

13. The method according to any one of claims 10 to 12, **characterized in that** the oxidative treatment agent (A) comprises the at least one amino acid in a total amount of 0.05 to 3 wt.%, preferably 0.1 to 2 wt.%, and particularly preferably 0.2 to 1.2 wt.%, each converted to the undissociated acid and based on the weight of the oxidative treatment agent (A).

14. The method according to any one of claims 11 to 13, **characterized in that** composition (B) is powdery and comprises at least one persulfate salt and/or at least one percarbonate salt.

## Revendications

1. Agent de traitement par oxydation pour fibres kératiniques, en particulier pour les cheveux, contenant
a) au moins un acide dicarboxylique ayant 2 à 10 atomes de carbone et/ou au moins un sel de cet ou ces acides,
b) au moins un acide aminé,
c) du peroxyde d'hydrogène, de préférence en une quantité de 0,5 à 13 % en poids, de manière davantage préférée de 1 à 7 % en poids, de manière particulièrement préférée de 2 à 6 % en poids et de manière tout particulièrement préférée de 3 à 4,5 % en poids (calculé en tant que H₂O₂ à 100 %), dans chaque cas par rapport au poids total de l'agent de traitement par oxydation selon l'invention, et
d) de l'eau, de préférence en une quantité de 20 à 95 % en poids, de préférence de 30 à 90 % en poids, de manière particulièrement préférée de 40 à 85 % en poids, de manière préférée entre toutes de 50 à 80 % en poids, dans chaque cas par rapport au poids total de l'agent de traitement par oxydation selon l'invention,
dans lequel l'agent de traitement par oxydation a un pH compris dans la plage allant de 2,5 à 6,5, de préférence dans la plage allant de 3 à 5,5, de manière particulièrement préférée dans la plage allant de 3,5 à 5,0, mesuré dans chaque cas à 20 °C.

2. Agent de traitement selon la revendication 1, **caractérisé en ce que** l'au moins un acide aminé est choisi parmi les acides aminés de formule (VI) 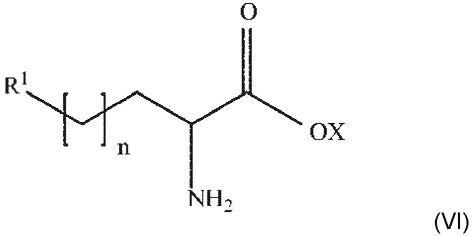 dans laquelle
X représente un atome d'hydrogène ou un cation monovalent ou divalent ;
n est égal à zéro, 1, 2 ou 3 ;
R¹ représente un radical choisi parmi un groupe amino, un groupe guanidine, un groupe (1*H*-imidazol-4-yl), un groupe amide d'acide carboxylique -CONH₂, un groupe 1*H*-indol-3-yl, un groupe thiol -SH et un groupe méthylsulfanyle -SCH₃, ou au moins un sel de cet acide aminé.

3. Agent de traitement selon la revendication 1 ou 2, **caractérisé en ce que** l'au moins un acide dicarboxylique ayant 2 à 10 atomes de carbone est choisi parmi l'acide maléique, l'acide fumarique, l'acide succinique, l'acide malique, l'acide oxalique, l'acide malonique, l'acide adipique, l'acide pimélique, l'acide subérique, l'acide azélaïque, l'acide sébacique, l'acide D-tartrique, l'acide L-tartrique, l'acide méso-tartrique, l'acide racémique, l'acide alpha-cétoglutarique, l'acide bêta-cétoglutarique et des mélanges de ces acides, est choisi de préférence parmi l'acide succinique, l'acide malique, l'acide maléique et l'acide fumarique, est choisi de manière particulièrement préférée parmi l'acide succinique.

4. Agent de traitement selon l'une quelconque des revendications 1 à 3, **caractérisé en ce que** l'au moins un acide dicarboxylique ayant 2 à 10 atomes de carbone est présent en une quantité totale de 0,1 à 5 % en poids, de préférence de 0,2 à 4 % en poids, de manière particulièrement préférée de 0,5 à 2 % en poids, convertis dans chaque cas en acide non dissocié et par rapport au poids de l'agent de traitement.

5. Agent de traitement selon l'une des revendications 2 à 4, **caractérisé en ce que** l'au moins un acide aminé de formule (VI) est choisi parmi l'arginine, la lysine, l'histidine, l'asparagine, la glutamine, la cystéine, la méthionine, le tryptophane et des mélanges de ceux-ci, de manière particulièrement préférée des mélanges d'arginine et de lysine.

6. Agent de traitement selon l'une quelconque des revendications 1 à 5, **caractérisé en ce que** l'au moins un acide aminé est présent en une quantité totale de 0,05 à 3 % en poids, de préférence de 0,1 à 2 % en poids, de manière particulièrement préférée de 0,2 à 1,2 % en poids, convertis dans chaque cas en acide non dissocié et par rapport au poids de l'agent de traitement.

7. Agent de traitement selon l'une quelconque des revendications 1 à 6, **caractérisé en ce qu'**au moins un agent chélatant est présent, de préférence en une quantité totale de 0,01 à 2,5 % en poids, de manière particulièrement préférée en une quantité totale de 0,1 à 1,5 % en poids, de manière préférée entre toutes en une quantité totale de 0,2 à 0,6 % en poids, dans chaque cas par rapport au poids de l'agent de traitement par oxydation.

8. Agent de traitement selon l'une quelconque des revendications 1 à 7, **caractérisé en ce qu'**il contient au moins un composé de formule générale (III), 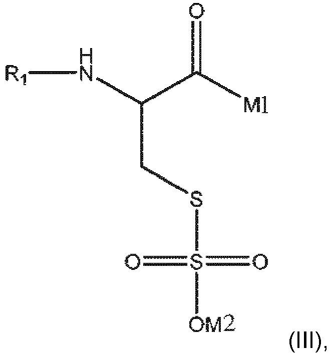 dans laquelle
R1 représente un atome d'hydrogène ou un élément structurel de formule (IV) 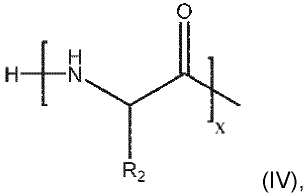 dans laquelle
x représente un nombre entier compris entre 1 et 100,
le radical R2 dans chacun des éléments structurels de formule (IV) peut être choisi indépendamment de l'élément structurel précédent de formule (IV),
R2 représente un atome d'hydrogène, un groupe méthyle, un groupe isopropyle, un groupe 2-méthylpropyle, un groupe 1-méthylpropyle, un groupe benzyle, un groupe 4-hydroxybenzyle, un groupe hydroxyméthyle, un groupe 1-hydroxyéthyle, un groupe 4-aminobutyle, un groupe 3-carbamimidamidopropyle, un groupe 2-carboxyéthyle, un groupe carboxyméthyle, un groupe 2-carbamoyléthyle, un groupe carbamoylméthyle, un groupe sulfanylméthyle, un groupe 2-(méthylsulfanyl)éthyle, un groupe 1H-imidazol-4-ylméthyle, un groupe 1H-indol-3-ylméthyle ou un groupe (sulfosulfanyl)méthyle, M1 représente le groupement -OM2 ou un élément structurel de formule (V) 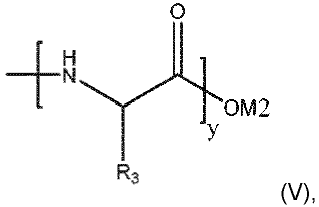 dans laquelle
y représente un nombre entier compris entre 1 et 100.
le radical R3 dans chacun des éléments structurels de formule (V) peut être choisi indépendamment de l'élément structurel précédent de formule (V),
R3 représente un atome d'hydrogène, un groupe méthyle, un groupe isopropyle, un groupe 2-méthylpropyle, un groupe 1-méthylpropyle, un groupe benzyle, un groupe 4-hydroxybenzyle, un groupe hydroxyméthyle, un groupe 1-hydroxyéthyle, un groupe 4-aminobutyle, un groupe 3-carbamimidamidopropyle, un groupe 2-carboxyéthyle, un groupe carboxyméthyle, un groupe 2-carbamoyléthyle, un groupe carbamoylméthyle, un groupe sulfanylméthyle, un groupe 2-(méthylsulfanyl)éthyle, un groupe 1H-imidazol-4-ylméthyle, un groupe 1H-indol-3-ylméthyle ou un groupe (sulfosulfanyl)méthyle, M2 représente un atome d'hydrogène, un équivalent d'un cation mono- ou polyvalent ou un ion ammonium (NH₄)⁺,
dans lequel de préférence un ou plusieurs composés de formule (III) susmentionnée sont présents en une quantité totale de 0,001 à 2,5 % en poids, de manière davantage préférée de 0,01 à 1,0 % en poids et de manière particulièrement préférée de 0,02 à 0,1 % en poids, dans chaque cas par rapport au poids de l'agent de traitement.

9. Agent de traitement selon l'une quelconque des revendications 1 à 8, **caractérisé en ce qu'**au moins un amide d'acide gras répondant à la formule (AMID-I) est présent, de préférence en une quantité totale de 0,1 à 10 % en poids, de manière particulièrement préférée de 0,2 à 5 % en poids, de manière préférée entre toutes de 0,4 et 2 % en poids, dans chaque cas par rapport au poids de l'agent de traitement, 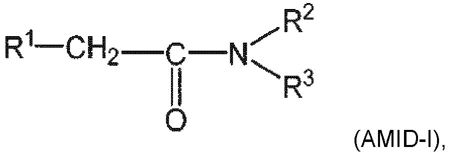 dans laquelle
- R¹ est choisi parmi les groupes alkyle en C4 à C22 linéaires et ramifiés, saturés ou insaturés, les groupes alkyle et alcényle étant non substitués ou pouvant être substitués par des atomes d'oxygène, des atomes d'azote, des groupes hydroxyle, des groupes éther, des groupes oxyalkylène, des groupes polyoxyalkylène, des groupes acide carboxylique, des groupes amine, des groupes amide, des groupes contenant de l'halogène, des groupes ester, des groupes siloxane ou des groupes polysiloxane, et parmi des groupes alkyle alcoxylés de formules : 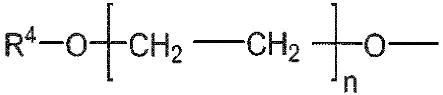 et 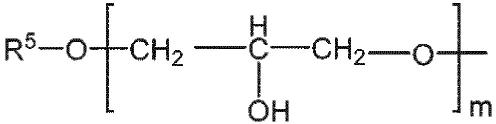 dans lesquelles :
- R⁴ et R⁵, qui peuvent être identiques ou différents, sont choisis parmi les groupes alkyle en C4 à C22 linéaires et ramifiés, saturés ou insaturés,
- n est un nombre entier compris dans la plage allant de 1 à 10, de préférence dans la plage allant de 1 à 4 et
- m est un nombre entier compris dans la plage allant de 1 à 6 et
- R² et R³, qui peuvent être identiques ou différents, sont chacun choisis indépendamment parmi H ; des groupes alkyle en C1 à C22 linéaires et ramifiés, saturés ou insaturés, les groupes alkyle et alcényle étant non substitués ou étant substitués par des atomes d'oxygène, des atomes d'azote, des groupes hydroxyle, des groupes éther, des groupes oxyalkylène, en particulier des groupes oxyéthylène, des groupes polyoxyalkylène, en particulier des groupes polyoxyéthylène, des groupes acide carboxylique, des groupes amine, des groupes alkylamine, en particulier des groupes alkylamine en C1 à C3, des groupes dialkylamine, en particulier des groupes di-alkylamine(en C1 à C3), des groupes 4,5-dihydroimidazolium substitués et non substitués, des groupes amide, des groupes contenant de l'halogène, des groupes ester, des groupes siloxane ou des groupes polysiloxane.

10. Procédé pour la coloration et/ou l'éclaircissement par oxydation de fibres kératiniques, en particulier des cheveux, dans lequel un agent de traitement selon l'une quelconque des revendications 1 à 9 est appliqué sur les fibres keratiniques, en particulier sur les cheveux, et est ensuite éventuellement rincé après une durée d'action de 0,1 à 60 minutes, de préférence de 1 à 50 minutes, de manière particulièrement préférée de 10 à 45 minutes, de manière préférée entre toutes de 30 à 45 minutes.

11. Procédé pour la coloration et/ou l'éclaircissement par oxydation de fibres kératiniques, en particulier des cheveux, comprenant les étapes de procédé suivantes :
I. préparer un agent de traitement par oxydation (A), contenant
a) au moins un acide dicarboxylique ayant 2 à 10 atomes de carbone et/ou au moins un sel de cet ou ces acides, qui est de préférence choisi parmi l'acide maléique, l'acide fumarique, l'acide succinique, l'acide malique, l'acide oxalique, l'acide malonique, l'acide adipique, l'acide pimélique, l'acide subérique, l'acide azélaïque, l'acide sébacique, l'acide D-tartrique, l'acide L-tartrique, l'acide méso-tartrique, l'acide racémique, l'acide alpha-cétoglutarique, l'acide bêta-cétoglutarique et des mélanges de ces acides, est choisi de manière particulièrement préférée parmi l'acide succinique, l'acide maléique et l'acide fumarique, est choisi de manière préférée entre toutes parmi l'acide succinique, ainsi que
b) au moins un acide aminé qui est choisi de préférence parmi les acides aminés de formule (VI) 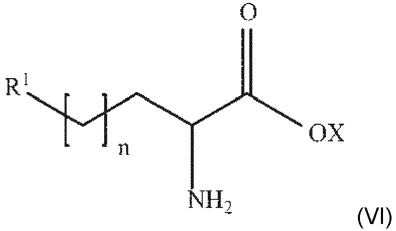 dans laquelle
X représente un atome d'hydrogène ou un cation monovalent ou divalent ;
n est égal à zéro, 1, 2 ou 3 ;
R¹ représente un radical choisi parmi un groupe amino, un groupe guanidine, un groupe (1*H*-imidazol-4-yl), un groupe amide d'acide carboxylique -CONH₂, un groupe 1*H*-indol-3-yl, un groupe thiol -SH et un groupe méthylsulfanyle -SCH₃, ou au moins un sel de cet acide aminé,
c) du peroxyde d'hydrogène,
d) de l'eau,
e) dans lequel l'agent de traitement par oxydation a un pH compris dans la plage allant de 2,5 à 6,5, de préférence dans la plage allant de 3 à 5,5, de manière particulièrement préférée dans la plage allant de 3,5 à 5,0, mesuré dans chaque cas à 20 °C,
f) éventuellement en plus au moins une substance, qui est choisie parmi
- des composés de formule générale (III), 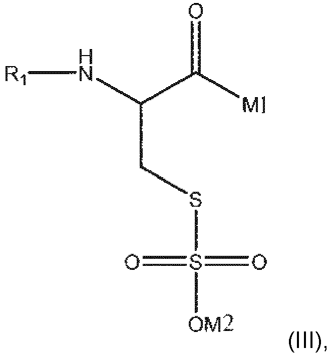 dans laquelle
R1 représente un atome d'hydrogène ou un élément structurel de formule (IV) 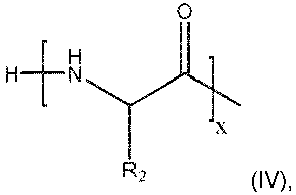 dans laquelle
x représente un nombre entier compris entre 1 et 100,
le radical R2 dans chacun des éléments structurels de formule (IV) peut être choisi indépendamment de l'élément structurel précédent de formule (IV),
R2 représente un atome d'hydrogène, un groupe méthyle, un groupe isopropyle, un groupe 2-méthylpropyle, un groupe 1-méthyl-propyle, un groupe benzyle, un groupe 4-hydroxybenzyle, un groupe hydroxyméthyle, un groupe 1-hydroxyéthyle, un groupe 4-aminobutyle, un groupe 3-carbamimidamidopropyle, un groupe 2-carboxyéthyle, un groupe carboxyméthyle, un groupe 2-carbamoyléthyle, un groupe carbamoylméthyle, un groupe sulfanylméthyle, un groupe 2-(méthylsulfanyl)éthyle, un groupe 1H-imidazol-4-ylméthyle, un groupe 1H-indol-3-ylméthyle ou un groupe (sulfosulfanyl)méthyle,
M1 représente le groupement -OM2 ou un élément structurel de formule (V) 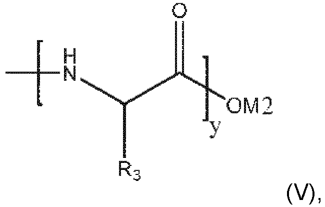 dans laquelle
y représente un nombre entier compris entre 1 et 100,
le radical R3 dans chacun des éléments structurels de formule (V) peut être choisi indépendamment de l'élément structurel précédent de formule (V),
R3 représente un atome d'hydrogène, un groupe méthyle, un groupe isopropyle, un groupe 2-méthylpropyle, un groupe 1-méthyl-propyle, un groupe benzyle, un groupe 4-hydroxybenzyle, un groupe hydroxyméthyle, un groupe 1-hydroxyéthyle, un groupe 4-aminobutyle, un groupe 3-carbamimidamidopropyle, un groupe 2-carboxyéthyle, un groupe carboxyméthyle, un groupe 2-carbamoyléthyle, un groupe carbamoylméthyle, un groupe sulfanylméthyle, un groupe 2-(méthylsulfanyl)éthyle, un groupe 1H-imidazol-4-ylméthyle, un groupe 1H-indol-3-ylméthyle ou un groupe (sulfosulfanyl)méthyle,
M2 représente un atome d'hydrogène, un équivalent d'un cation mono- ou polyvalent ou un ion ammonium (NH₄)⁺,
- au moins un agent chélatant, de préférence en une quantité totale de 0,01 à 2,5 % en poids, de manière particulièrement préférée en une quantité totale de 0,1 à 1,5 % en poids, de manière préférée entre toutes en une quantité totale de 0,2 à 0,6 % en poids, dans chaque cas par rapport au poids de l'agent de traitement par oxydation (A), et
- au moins un amide d'acide gras répondant à la formule (AMID-I), de préférence en une quantité totale de 0,1 à 10 % en poids, de manière particulièrement préférée de 0,2 à 5 % en poids, de manière préférée entre toutes de 0,4 et 2 % en poids, dans chaque cas par rapport au poids de l'agent de traitement par oxydation (A), 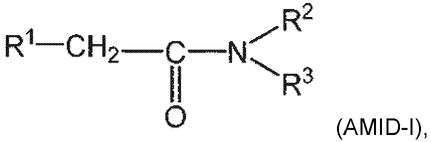 dans laquelle
R¹ est choisi parmi les groupes alkyle en C4 à C22 linéaires et ramifiés, saturés ou insaturés, les groupes alkyle et alcényle étant non substitués ou pouvant être substitués par des atomes d'oxygène, des atomes d'azote, des groupes hydroxyle, des groupes éther, des groupes oxyalkylène, des groupes polyoxyalkylène, des groupes acide carboxylique, des groupes amine, des groupes amide, des groupes contenant de l'halogène, des groupes ester, des groupes siloxane ou des groupes polysiloxane, et parmi des groupes alkyle alcoxylés de formules : 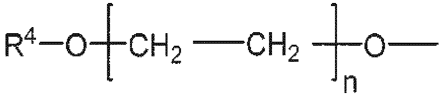 et 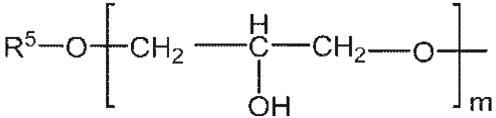 dans lesquelles :
R⁴ et R⁵, qui peuvent être identiques ou différents, sont choisis parmi les groupes alkyle en C4 à C22 linéaires et ramifiés, saturés ou insaturés, n est un nombre entier compris dans la plage allant de 1 à 10, de préférence dans la plage allant de 1 à 4 et m est un nombre entier compris dans la plage allant de 1 à 6, et
R² et R³, qui peuvent être identiques ou différents, sont chacun indépendamment l'un de l'autre choisis parmi H ; des groupes alkyle en C1 à C22 linéaires et ramifiés, saturés ou insaturés, les groupes alkyle et alcényle étant non substitués ou étant substitués avec des atomes d'oxygène, des atomes d'azote, des groupes hydroxyle, des groupes éther, des groupes oxyalkylène, en particulier des groupes oxyéthylène, des groupes polyoxyalkylène, en particulier des groupes polyoxyéthylène, des groupes acide carboxylique, des groupes amine, des groupes alkylamine, en particulier des groupes alkylamine en C1 à C3, des groupes dialkylamine, en particulier des groupes dialkylamine(en C1 à C3), des groupes 4,5-dihydroimidazolium substitués et non substitués, des groupes amide, des groupes contenant de l'halogène, des groupes ester, des groupes siloxane ou des groupes polysiloxane,
II. préparer une composition (B), contenant
g) au moins un agent d'alcalinisation, choisi parmi l'hydroxyde d'ammonium, la monoéthanolamine et les silicates de sodium ainsi que des mélanges de ceux-ci,
h) éventuellement de l'eau et
i) éventuellement au moins un précurseur de colorant d'oxydation et/ou au moins un colorant direct,
la composition (B) ayant de préférence un pH compris dans une plage allant de 6,5 à 12,0, de préférence de 8 à 11,5, de manière particulièrement préférée de 8,5 à 11,0, mesuré dans chaque cas à 20 °C,
III. mélanger les compositions (A) et (B) l'une avec l'autre, le rapport pondéral entre (A) et (B) étant compris de préférence dans la plage allant de 10:1 à 1:4, de manière préférée dans la plage allant de 5:1 à 1:3, de manière particulièrement préférée dans la plage allant de 4:1 à 1:2, de manière préférée entre toutes dans la plage allant de 2:1 à 1:1, et immédiatement après
IV. appliquer le mélange de (A) et (B) sur les fibres kératiniques, en particulier sur les cheveux, et
V. rincer après une durée d'action de 0,1 à 60 minutes, de préférence de 1 à 50 minutes, de manière particulièrement préférée de 10 à 45 minutes, de manière préférée entre toutes de 30 à 45 minutes,
VI. réaliser éventuellement d'autres traitements capillaires tels que le brushing, le conditionnement et/ou le séchage.

12. Procédé selon la revendication 10 ou 11, **caractérisé en ce que** l'agent de traitement par oxydation (A) contient l'au moins un acide dicarboxylique ayant 2 à 10 atomes de carbone en une quantité totale de 0,1 à 5 % en poids, de préférence de 0,2 à 4 % en poids, de manière particulièrement préférée de 0,5 à 2 % en poids, dans chaque cas converti en acide non dissocié et par rapport au poids de l'agent de traitement (A).

13. Procédé selon l'une quelconque des revendications 10 à 12, **caractérisé en ce que** l'agent de traitement par oxydation (A) contient l'au moins un acide aminé en une quantité totale de 0,05 à 3 % en poids, de préférence de 0,1 à 2 % en poids, de manière particulièrement préférée de 0,2 à 1,2 % en poids, dans chaque cas converti en acide non dissocié et par rapport au poids de l'agent de traitement par oxydation (A).

14. Procédé selon l'une quelconque des revendications 11 à 13, **caractérisé en ce que** la composition (B) est pulvérulente et contient au moins un sel de persulfate et/ou au moins un sel de percarbonate.
